# EUROPEAN PATENT APPLICATION

(11) **EP 1 792 979 A1**
(43) Date of publication of application: **06.06.2007**
(21) Application number: 05026248.4
(22) Date of filing: 01.12.2005
(51) Int. Cl.: C12N 5/06

(54) **Cell culture system for the enrichment and expansion of stem cells**

(71) Applicant: Stiftung Caesar Center of Advanced European Studies and Research, 53175 Bonn (DE)
(72) Inventor: Brenner, Norbert, 53227 Bonn (DE); Royer, Hans-Dieter, 40225 Düsseldorf (DE); Hoffmann, Karl-Heinz, 53177 Bonn (DE)
(74) Representative: Müller Fottner Steinecke

(57) **Abstract**

The present invention relates to methods and compositions concerning the isolation of stem cells. In particular, methods are provided which allow the enrichment and isolation of stem cells in a mixture of stem cells and non-stem cells, wherein the isolated stem cells are substantially free from contaminating non-stem cells, for example differentiated cells. Furthermore, the present invention provides serum-free media which support the growth of stem cells, in particular adult stem cells and tumor stem cells in culture. The present invention also provides multi- and pluripotent stem cells which can be used for various applications including transplantation, toxicity screening, expression of recombinant proteins and the production of transgenic animals. The stem cells of the present invention are particular useful for investigating stem cell function, in particular of tumor stem cells using microarray technology.

## Description

### Field of the invention

The present invention generally concerns a cell culture system which allows the growth and enrichment of stem cells and their separation from non-stem cells. More specifically, the present invention relates to novel methods and compositions that allow maintaining selectively stem cells including adult stem cells and tumor stem cells in liquid suspension either as single cells or spheroids, thereby enriching and separating said stem cells from differentiated and differentiating cells. The present invention particularly relates to methods and cell culture media for the enrichment and isolation of adult stem cells and cancer stem cells. The present invention further relates to the use of the stem cells obtained in accordance with the method of the present invention for tissue engineering, gene expression profiling and for identifying or assaying compounds, for example cytostacics, therapeutic agents, hormones, blocking compounds and for use in toxicity screening. In particular, the present invention relates to the use of the cell culture system and stem cells of the present invention in drug discovery and microarray technology.

### Background of the invention

Stem cells, which nowadays can be isolated almost from any organ, have to date been expanded using very different and in many cases organ-specific cell culture systems. Some examples for specific systems are embryonic stem cells (Thomson et al., Science 282 (1998), 1145-1147, embryonic germ (EG) cells (Gearhart, Proc. Natl. Am. Soc. 98 (2001), 113-118), teratocarcinoma stem cells (Andrews, Biochim. Biophys. Acta 948 (1988), 17-36), neuronal stem cells (Gage, Science 287 (2000), 1433-1438), bone marrow stem cells (Verfaillie, Nature 418 (2002), 41-49), hematopoietic stem cells and others. According to the actual state of the art, the selection of stem cells is difficult, since no constant surface marker being common to all stem cells is known, which can be used for their isolation. Stem cells are in principle only a small subfraction within different states of differentiation of somatic cells and carry different markers according to their origin.

The variety of the culture systems which are applied to stem cells derived from different regions of the body, results in a bad comparability of the stem cells with each other. Depending on the culture conditions the cells present different markers and show restricted differentiability as well as other behavior patterns. A main idea which finally resulted in the method of the present invention was that in an environment enabling the maintenance of the earliest possibly state of development all stem cells exhibit their highest potential for differentiation. Additionally, due to the uniform culturing conditions, they should also present unique markers, which is important for the comparability using new chip technologies. Thus, the differences can thereby be distinguished much better.

Embryonic stem cell technology may serve as an example for such a system, since said cells correspond to the earliest state of development. Pluripotent teratocarcinoma cells which behave like embryonic stem cells are highly appropriate for the adjustment of the culturing conditions. At the same time, those cells are exemplary for the search of cancer stem cells.

Cancer is a disease in which the growth and differentiation of cells is no longer controlled. According to the current state of the art, there is increased evidence that cancer originates from stem cells. In cancer diseases it could have been shown that the cancerous tissue contains cell types identical to those of the surrounding tissue. Thereby, the similarity of the stem cells and precursor cells derived from the cancerous tissue to the stem cells of healthy tissue is striking. In particular, immature cells of different differential states originated, which do not reach the finite state of differentiation and thereby still proliferate. As described hereinbefore, there is a direct correlation between the duration of a cell cycle and the differentiation of cancer cells and stem cells, both utilizing the same signaling pathways (for example WNT, gp130, EGF signaling). Thus, the balance between proliferation and differentiation is disturbed (Pierce, Am. J. Pathol. 77 (1974), 103-118).

It is known that embryonic stem cells develop teratocarcinomas during transplantation. This, as well as many other evidences, like for example, similar markers, transcription factors or regulation mechanisms confirm the theory that cancer originates from stem cells. During transplantation of cancer cells, only a small amount of cells called "clonogenic" show tumorigenic potential (Greaves, J. Cell Physiol. 1 (Suppl. 1982), 113-125). Those cells probably correspond to the cancer stem cells causing metastazation in patients suffering from cancer (Reya, Nature 414 (2001), 105-111).

A widespread phenomenon and most of all the major clinical problem in the treatment of cancer diseases is the formation of multiresistance. Here also common properties of stem cells and cancer cells can be observed, since both are able to activate protection mechanisms against harmful environmental influences. Since one function of stem cells is to repair damaged tissue, it is reasonable that they protect themselves in case of stress in order to maintain this function. Exactly for this reason also for example ABC transporters are activated, being proteins causing the detoxification by discharging the toxic substances out of the cell. The same mechanisms cause the resistance against chemotherapeutics in case of cancer diseases. By this, the enormous relevance of the enrichment of cancer stem cells for further investigations concerning their resistance is obvious. To date, in vitro chemosensitivity tests show a bad correlation to the in vivo situation, making the success of a therapy insufficiently predictable. The resistant cancer stem cells causing the "relapse" have to date not been in the focus of investigation, although their clinical relevance is enormous.

According to the actual state of the art, cancer stem cells can only be isolated from tumors using an enormous mechanical effort. This isolation was often carried out using a cell sorter leading to the isolation of so-called "side population cells" (SP cells). Although additionally, those cells can also be isolated using magnetic beads, which target the desired cells by antibodies, the existence of the corresponding markers of the target cells has to be known beforehand. Those cells form a subfraction of the tumor, but it is not entirely clear whether all cancer stem cells correspond to the selection criterion (discharging of e.g. Hoechst dye via PGP or a special marker).

Therefore, an object of the present invention is that stem cells as well as the tumorigenic cancer stem cells can be made much more easily accessible by an in vitro culture system. It should not be necessary to find special markers and to use complex equipment. This should simplify the isolation of stem cells and cancer stem cells and enable at the same time their expansion in culture. In many cases the material is not sufficient to carry out direct tests (chips). In addition, the present invention aims at the selection of the stem cells and cancer stem cells, as other cell types terminally differentiate and become apoptotic in suspension or simply stop proliferation. The suspended stem cells and spheres or spheroids, respectively, can be easily separated from the remaining cells and then be further investigated. This may be used to get new insights in cancer research, for simplified and clearer diagnostics and for a specific treatment of patients.

Conventional cell culture media, during their production concerning the composition of the substances and their concentration have been adapted to the original tissue from which the cells intended to be cultivated were extracted or are emerged from testing different basic media and supplements. Since cells react to external signals, they adapt to the conditions of the cell culture system and show corresponding behavior patterns, as imposed by the system. Since cells intensively communicate with their environment, every change of condition leads to complex reactions of the cells.

For example a cell culture system which corresponds to neuronal patterns leads to an adaptation of the multipotent cells so that they lose their multipotency and specialize during their differentiation to neuronal tissue types. This results in tissue-specific potentials of differentiation and the loss of the potential to form different tissues (loss of multipotency) (Schuldiner, Proc. Natl. Am. Soc. 21 (2000), 11307-11312).

In conventional monolayer cultures the cells adhere to a plastic surface and have contact to each other only at very small areas. The informational exchange between the cells is thereby blocked and by the limitation to the plastic surface and to the medium the exchange is altered compared to native tissue. Thus, this brings about different polarities and special orientations in the cells, having an unnatural influence on the cells' behavior in the cell culture system. In three-dimensional cell culture systems said cells show a different behavior (Bissell, Nature 424 (2003), 870-872).

In conventional cell culture systems undifferentiated proliferation of stem cells is additionally hampered due to the asymmetric division, wherein from one stem cell one further stem cell and one Transient Amplifying (TA) cell originate. The TA cell has a much stronger proliverative activity and exhibits an advanced differentiation and thereby is restricted concerning the potential to form different cell types. The accelerated growth of the TA cells leads to the overgrowing of the slower proliferating multipotent stem cells and represses them. It is depending on the cell culture conditions, whether the stem cells undergo a symmetric or asymmetric division. The differentiating cells produce in turn growth factors and signal substances which may stimulate the stem cells to differentiate.

In conventional cell culture systems the cells are stimulated to divide by the addition of serum. Using serum means making a compromise between the stimulation of growth and the stimulation of differentiation. Serum contains a plurality of biologically active substances like for example hormones and growth factors. Said substances stimulate the cells to divide, however also provide a lot of stimuli for differentiation. The composition of the serum is hardly controllable and is subject to big oscillations. A further disadvantage is the origin from animals, since with the serum also pathogens can be transmitted to humans.

The use of "feeder cells" also brings about a high risk of contamination, although they provide the necessary signals for the stem cells. The feeder cells "contaminate" the stem cells by their presence or e.g. by possibly therein contained viruses. This hampers the application of said cells in the human system, especially for transplantation purposes. The use of human fibroblasts as feeder cells is also described in the literature. Experiments to proliferate for example embryonic stem cells with conditioned medium of those cells is discussed in literature controversially (Xu et al., Nat. Biotechnol. 19 (2001), 971-974; Richards et al., Nat. Biotechnol. 20 (2002), 933-936). Additionally, the composition of a conditioned medium is object to oscillations and by the plurality of the secreted substances the important factors are hardly recognizable. In comparing investigations this also can lead to disorders.

The solution to the above described technical problems is achieved by providing the embodiments characterized in the claims and described further below.

### Summary of the invention

The present invention is directed to a cell culture system in which pluripotent and multipotent stem cells as well as cancer stem cells are selectively enriched and expanded, maintaining their pluripotency and multipotency, respectively. In particular, the present invention employs passaging suspended cells in liquid media to enrich the cells for non-differentiated cells as opposed to differentiated cells. Additionally the system is ideal as a study model for cancer cells, since cancer cells in this culture system show almost the same behavior as those in a native tumor.

In particular, it is an object of the present invention to provide a feeder layer-free culture system for stem cells, especially for adult stem cells and cancer stem cells, based on the anchorage dependency of non-stem cells, the mobility of stem cells and the appropriate choice of the culture surface. More specifically, a feeder layer- and serum-free culture medium is provided supplemented with about 20% serum replacement, basic fibroblast growth factor (bFGF), optionally further supplemented with a combination of growth factors including transforming growth factor β1 (TGFβ1) and additional medium additives. Stem cells of various origin grown in these conditions maintain all stem cell features after prolonged culture, including the developmental potential to differentiate into representative tissues of the three embryonic germ layers, unlimited and undifferentiated proliferative ability, and maintenance of normal karyotype (or abnormal karyotype in most cases of tumor stem cells). The cell culture system presented here has at least three major advantages: (1) application of a well-defined culture system to any kind of stem cells; (2) reduced exposure of in particular human stem cells to animal pathogens; and (3) its application for the isolation and maintenance of tumor stem cells and non-hematopoietic cells. The feeder layer- and serum free culture system reported here aims at facilitating research practices and providing a safer alternative for future clinical applications of stem cells.

The present invention also exploits the properties of stem cells to detach actively from an adherence-mediating culture surface and pass over in suspension, when the surface is coated with L-amino acids like poly L-lysine or poly L-ornithine or others. This is because stem cells compared to differentiated cells are able to secrete enzymes, for example proteases, in particular metalloproteases. Since those enzymes (metalloproteases) do not work on D-amino acids, said amino acids are not an appropriate coating material for the selection of stem cells. This step can be used as an upstream step before cells are further cultivated in the culture system to accelerate the enrichment of stem cells in suspension and the retention of adhered differentiated cells. By passaging of the suspension in a freshly coated cell culture flask a fast enrichment of stem cells in suspension is achieved.

In some embodiments of the present invention, the stem cells are utilized themselves as being therapeutic, although in other embodiments the stem cells are employed as a vehicle for the delivery of a therapeutic agent. In further embodiments, the stem cells are both therapeutic and provide a therapeutic agent.

As demonstrated in the examples, the cell culture system of the present invention is applicable to most if not all types of stem cells contrary to previous systems that hitherto had to be specifically adapted for the stem cell concerned.

Hence, in one aspect the present invention relates to a feeder layer- and serum-free as well as non-conditioned culture medium and culture of stem cells, respectively.

Furthermore, the present invention relates to culture containers and surfaces for use in the culture system of the present invention, for example flasks, dishes, multiwell plates, roller bottles, stirred vessels (spinner flasks), bioreactors, or fermentors. In one embodiment of the present invention, said container may be pre-filled with a cell culture medium of the present invention.

In a further aspect, the present invention relates to the use of a Wnt-pathway activating or upregulating substance, most preferably E-cadherin for effecting symmetric cell division of stem cells and suppressing the generation of Transit Amplifying (TA) cells.

It is another object of the present invention to provide a method for screening compounds which affect proliferation, differentiation or survival of stem cells and/or differentiated derivatives thereof, comprising preparing stem cells according to the method of the invention; contacting a sample comprising said stem cells with at least one compound and monitoring the effect of the compound on proliferation, differentiation or survival of stem cells and/or differentiated derivatives thereof comprised within the sample.

According to another aspect, the invention relates to a therapeutic agent identified or isolated according to the method of the invention, which agent hitherto has not been known or used as a therapeutic agent, as well as a pharmaceutical composition comprising the therapeutic agent, and optionally a pharmaceutically acceptable carrier.

In yet another embodiment, the present invention relates to a kit for use in the method of the present invention comprising the culture medium, the cell culture system, as characterized in the detailed description of the present invention or culture media components thereof, growth factors, selectable markers, reference samples, microarrays, chelating substances, elutriation media, cell suspending media, blocking media, washing media, and components of said media, cell culture containers, vectors, nucleic acid probes, and/or a bioreactor. Preferably, the cell culture kit of the present invention comprises written instructions for how to perform any one of the cell culture and screening methods described herein.

Additionally, the present invention concerns the use of one or more stem cells or differentiated cells obtained by method of the invention, for loss of function assays of specific genes, gain of function assays of exogenous genes, analysis and screening of putative drugs, pharmacological assays, microarray systems, establishment of model systems for pathological cell functions, testing of anti-tumor agents, for investigating differentiation in vitro and/or in vivo, for tissue engineering, for compound testing or screening in pharmaceutical development, for activity or toxicological testing or therapeutic cell replacement strategies including but not limited to transplantation.

Besides the isolation and characterization of (cancer) stem cells, the methods of the invention can also be used for toxicological, mutagenic, and/or teratogenic in vitro tests, because people suffering from a disease are more vulnerable to intoxication and susceptible to side effects of pharmaceuticals, nutritions or any other compounds that one gets in contact with. Since the cells and tissue obtained in accordance with the present invention resemble more closely the in vivo situation, the results obtained by the toxicological assays of the present invention are expected to correlate to in vivo toxicity of the tested compounds as well.

In vitro tests for the sensitivity of cancer cells to chemotherapeutic agents conducted to date, revealed a bad correlation between tests and in vivo situation, because of which the therapeutic success using a certain chemotherapeutic was only insufficiently predictable. Furthermore, until now, only little attention was paid to the resistant cancer stem cells although they are assumed to cause the "relaps", i.e. the recurrence of cancer. Thus, it is a further object of the present invention to provide a cell culture system which allows the enrichment of pure cancer stem cells to make them accessible for investigation, since they are of extreme clinical relevance in view of fighting cancer diseases.

Other embodiments of the invention will be apparent from the description that follows.

### Brief description of the drawings

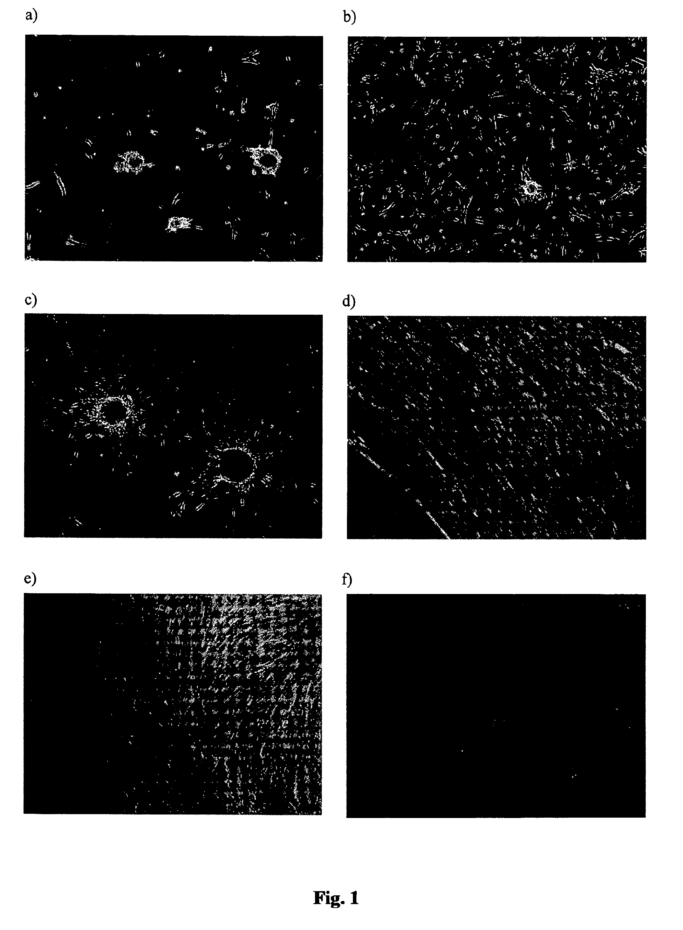

- **Fig. 1**:: illustrates the growth, formation of certain spatial arrangements and differentiation of cells obtained from the dental sac after cultivation in the cell culture system of the invention. Images were obtained using a phase-contrast microscope.
a) shows the formation of spheres in the primary culture of cells obtained from the dental sac after cultivation for three weeks.
b) shows the formation of new spheres, after the cells having been passaged once and seeded into the same cell culture system.
c) shows unique, fibroblastal morphologies of cells from different spheres, after being removed from the suspension, enzymatically digested, further passaged and seeded onto matrigel.
d) shows differentiation of the spheres into adipocytes induced by the addition of DMEM containing 10 % bovine serum, 1 mM L-glutamine, 100 µg/ml isobutylmethylxanthine, 60 mM indomethacin, 1 µM dexamethasone and 10 µg/ml insulin. Adipocytes were stained with Oil Red O.
e) shows differentiation into osteoblasts, induced by the addition of DMEM containing 10 % bovine serum, 1 mM L-glutamine, 10 mM β-glycerophosphate, 0.2 mM ascorbic acid and 0.1 µM dexamethasone. Osteoblasts were stained with alizarine red.
f) shows differentiation into neuronal cell types, induced by the addition of 1 µM all-trans retinoic acid in the neurobasal medium with B27 supplement and 1% L-glutamine.

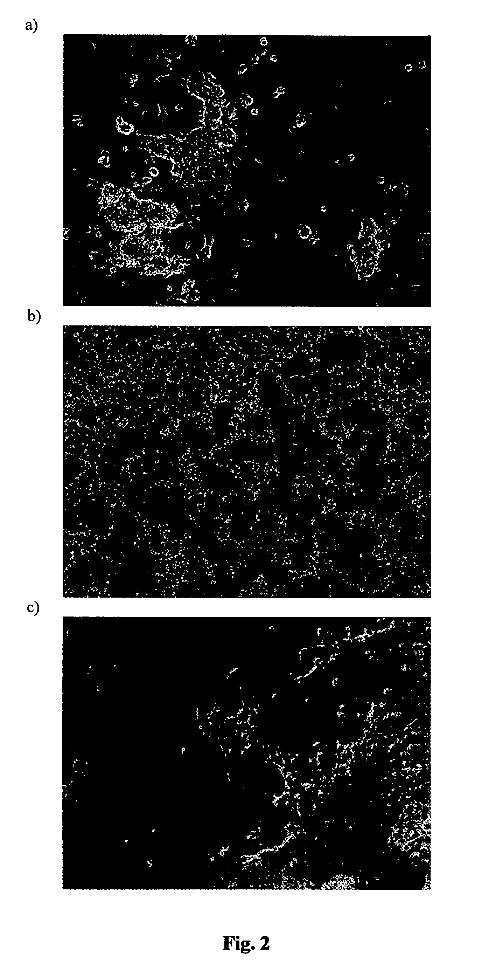

- **Fig. 2**:: illustrates the growth, formation of certain spatial arrangements and differentiation of NTERA-2 cl.D1, NT2/D1, teratocarcinoma cells after cultivation in the cell culture system of the invention. Images were obtained using a phase-contrast microscope.
a) shows the formation of spheres in the primary culture of the teratocarcinoma cells, after cells have been cultivated for 10 days.
b) shows the formation of new spheres, after the cells having been passaged once and seeded into the same system.
c) shows differentiation of the spheres into neuronal cells, after stimulation with ATRA.

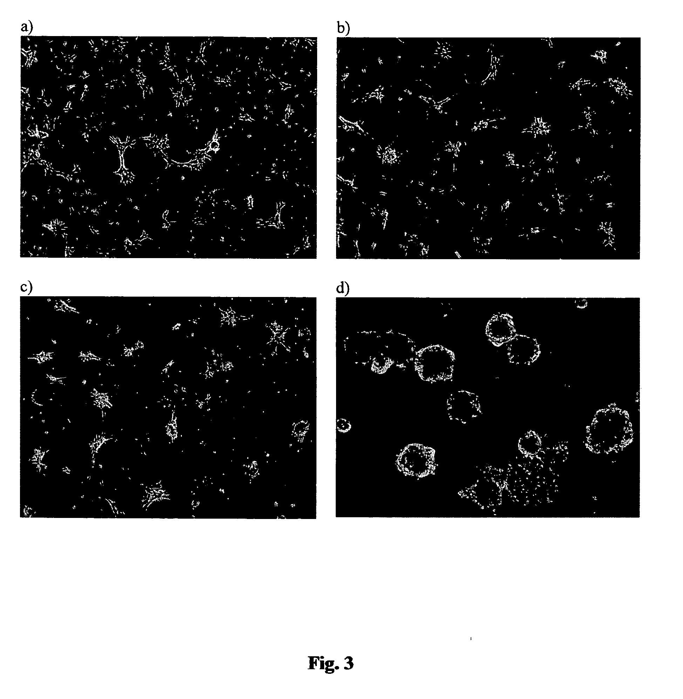

- **Fig. 3**:: illustrates the growth, formation of certain spatial arrangements and differentiation of mesenchymal stem cells after cultivation in the cell culture system of the invention. Images were obtained using a phase-contrast microscope.
a) shows first dense germs of spheres in the primary culture of mesenchymal stem cells after cells have been cultivated for 18 hours.
b) shows fortified presence of those germs of spheres after a cultivation time of 36 hours.
c) shows the formation of "mature" spheres after cultivation time of 60 hours.
d) shows the formation of new spheres, after the cells having been passaged once and seeded into the same cell culture system.

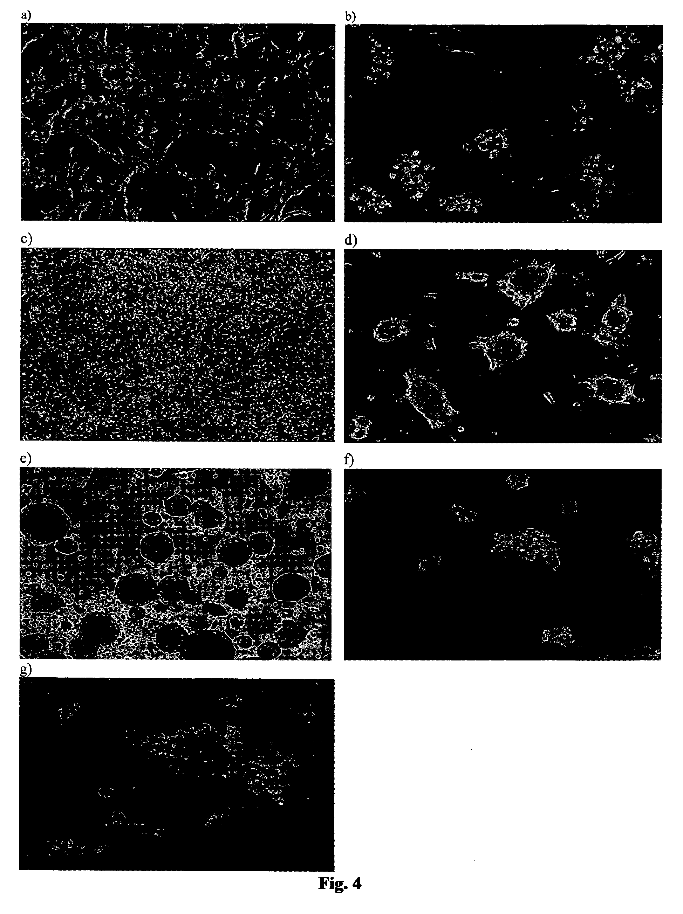

- **Fig. 4**:: illustrates the growth, formation of certain spatial arrangements and differentiation of HBL-100 cells, breast cells isolated from breast milk and after cultivation in the cell culture system of the invention. Images were obtained using a phase-contrast microscope.
a) shows adherent and non-adherent cells in the primary culture of HBL-100 cells after they have been cultivated for 18 hours. Microscopy was carried out at a magnification of 100.
b) shows beginning of slight adherence of the cells to each other after a cultivation time of 36 hours. Microscopy was carried out at a magnification of 400.
c) shows an increased amount of cells being in suspension beginning after a cultivation time of 60 hours. Microscopy was carried out at a magnification of 100.
d) shows the formation of new spheres, after cells were cultivated for two days, passaged once and seeded into the same cell culture system. Microscopy was carried out at a magnification of 100.
e) shows continued formation of spheres, after continued cultivation and passaging of the cells. Microscopy was carried out at a magnification of 100.
f) shows differentiation into neuronal cells, six days after induction with ATRA. Microscopy was carried out at a magnification of 400.
g) shows differentiation into neuronal cells, six days after induction with ATRA. Microscopy was carried out at a magnification of 200.

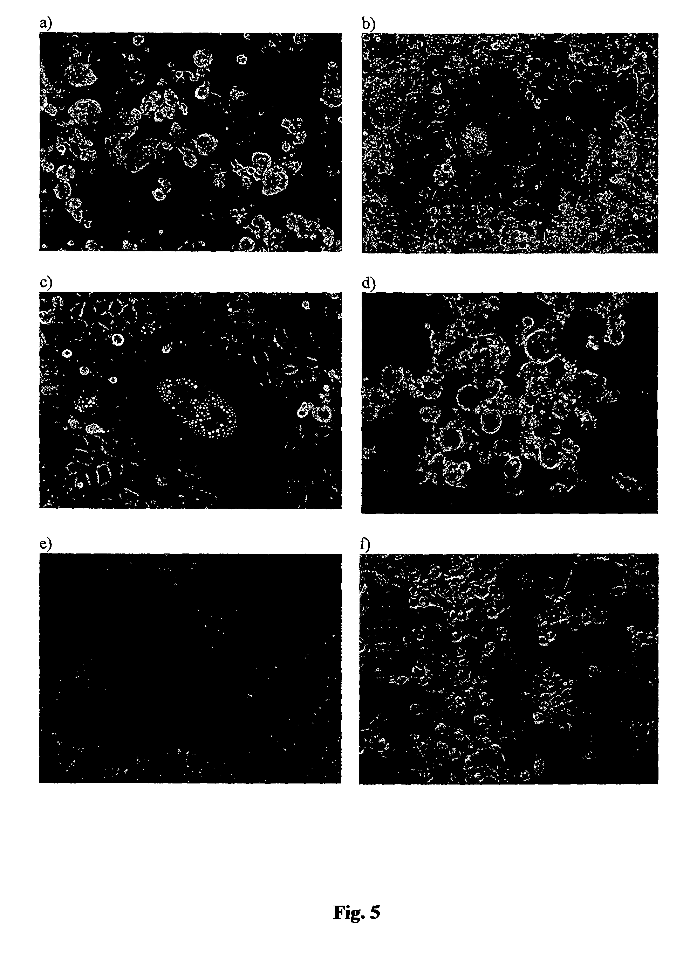

- **Fig. 5**:: illustrates the growth, formation of certain spatial arrangements and differentiation of MCF-7 cells, cells of a breast cancer cell line after cultivation in the cell culture system of the invention. Images were obtained using a phase-contrast microscope.
a) shows the splitting up of the cell culture after two days incubation in adherent and non-adherent cells, wherein the non-adherent cells show close-packed and hollow acinaric spheres. Microscopy was carried out at a magnification of 100.
b) shows the arise of polynucleal cells after extended cultivation time. Microscopy was carried out at a magnification of 100.
c) see b) Microscopy was carried out at a magnification of 400.
d) shows the enrichment of dark, massive spheres after long-time cultivation of the cells. Microscopy was carried out at a magnification of 200.
e) shows differentiation into neuronal cells, three days after induction with ATRA. Microscopy was carried out at a magnification of 400.
f) shows differentiation into neuronal cells, seven days after induction with ATRA. Microscopy was carried out at a magnification of 200.

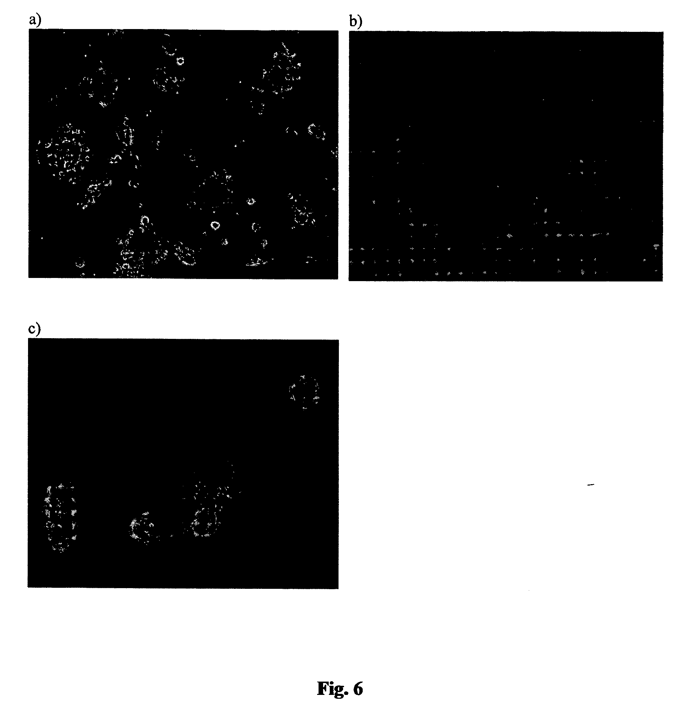

- **Fig. 6**:: illustrates the growth, formation of certain spatial arrangements and differentiation of cells taken from an ovarian carcinoma after cultivation in the cell culture system of the invention. Images were obtained using a phase-contrast microscope.
a) shows the formation of spheres in the primary culture of the ovarian carcinoma cells after they have been cultivated for three weeks. Microscopy was carried out at a magnification of 100.
b) shows the formation of new spheres, after one month and the cells having been passaged and seeded into the same system. Microscopy was carried out at a magnification of 400.
c) shows the adherence of "flakes" of small stem cells to a big differentiated cell. Microscopy was carried out at a magnification of 400.

### Definitions

For the purposes of this description, the term "stem cell" can refer to either stem cell or germ cell, for example embryonic stem (ES) and germ (EG) cell, respectively, but also including adult stem cells and tumor stem cells. Minimally, a stem cell has the ability to proliferate and form cells of more than one different phenotype, and is also capable of self-renewal, either as part of the same culture, or when cultured under different conditions. Embryonic stem cells are also typically telomerase positive and OCT-4 positive. Telomerase activity can be determined using TRAP activity assay (Kim et al., Science 266 (1997), 2011), using a commercially available kit (TRAPeze(R) XK Telomerase Detection Kit, Cat. s7707; Intergen Co., Purchase N.Y.; or TeloTAGGG(TM) Telomerase PCR ELISAplus, Cat. 2,013,89; Roche Diagnostics, Indianapolis). hTERT expression can also be evaluated at the mRNA level by RT-PCR. The LightCycler TeloTAGGG(TM) hTERT quantification kit (Cat. 3,012,344; Roche Diagnostics) is commercially available for research purposes.

In accordance with the present invention, the term embryonic stem (ES) cell includes any multi- or pluripotent stem cell derived from pre-embryonic, embryonic, or fetal tissue at any time after fertilization, and have the characteristic of being capable under appropriate conditions of producing progeny of several different cell types that are derivatives of all of the three germinal layers (endoderm, mesoderm, and ectoderm), according to a standard art-accepted test, such as the ability to form a teratoma in 8-12 week old SCID mice.
"Embryonic germ cells" or "EG cells" are cells derived from primordial germ cells. The term "embryonic germ cell" is used to describe cells that exhibit an embryonic pluripotent cell phenotype. The terms "human embryonic germ cell (EG)" or "embryonic germ cell" can be used interchangeably herein to describe mammalian, preferably human cells, or cell lines thereof, that exhibit a pluripotent embryonic stem cell phenotype as defined herein. Thus, EG cells are capable of differentiation into cells of ectodermal, endodermal, and mesodermal germ layers. EG cells can also be characterized by the presence or absence of markers associated with specific epitope sites identified by the binding of particular antibodies, as well as the absence of certain markers as identified by the lack of binding of certain antibodies.

"Progenitor cells" as used herein, refers to cells that are not fully mature or differentiated, but are less developmentally primitive than stem cells and which are progeny of a less differentiated stem cell. In contrast to stem cells, progenitor cells are not believed to be typically indefinitely self-replicating and, functionally, are not typically capable of long-term clonogenicity in immunodeficient animals in xenograft assays.

Furthermore, stem cells may be defined as self-renewing cells that undergo symmetric and asymmetric divisions to self-renew or differentiate into multiple kinds of differentiated progeny (Lin and Schagat, Trends Genet. 13 (1997), 33-39; Morrison et al., Cell 88 (1997), 287-298; Bums and Zon, Dev. Cell 3 (2002), 612-613). There are at least three types of stem cells: Totipotent, pluripotent and multipotent. A single totipotent stem cell can grow into an entire organism. Pluripotent stem cells cannot grow into a whole organism, but they can become any other cell of a particular germ layer, such as ectoderm, mesoderm or endoderm. Multipotent (also referred to as unipotent) stem cells can become any cells of a given tissue derived from one of the germ layers.

"Pluripotent" refers to cells that retain the developmental potential to differentiate into a wide range of cell lineages including the germ line. The terms "embryonic stem cell phenotype" and "embryonic-like stem cell" also are used interchangeably herein to describe cells that are undifferentiated and thus are pluripotent cells and that preferably are capable of being visually distinguished from other adult cells of the same animal.

Included in the definition of ES cells are embryonic cells of various types, exemplified by human embryonic stem cells, described by Thomson et al. (Science 282 (1998), 1145); embryonic stem cells from other primates, such as Rhesus stem cells (Thomson et al., Proc. Natl. Acad. Sci. USA 92 (1995), 7844), marmoset stem cells (Thomson et al., Biol. Reprod. 55 (1996), 254) and human embryonic germ (hEG) cells (Shamblott et al., Proc. Natl. Acad. Sci. USA 95 (1998), 13726). Other types of pluripotent cells are also included in the term. Any cells of mammal origin that are capable of producing progeny that are derivatives of all three germinal layers are included, regardless of whether they were derived from embryonic tissue, fetal tissue, or other sources. However, it is preferred not to use ES cells that are derived from a malignant source.

In order to avoid the use of for example human embryos as donors for stem cells, which however seems to be justifiable at least under certain circumstances, it may even be possible to employ transgenic non-human animals, in particular mammals, as source for embryonic stem cells. For example, compositions and methods for making transgenic swines to be used as xenograft donors are described in US patent No. 5,523,226. Likewise, international application WO97/12035 describes methods of producing transgenic animals for xenotransplantation. Furthermore, immunologically compatible animal tissues, suitable for xenotransplantation into human patients, are described in international applicationWO01/88096. Methods for making embryonic germ cells from porcine are described for example in US patent US-A-6,545,199. Cells, immunologically compatible with humans can also be employed for purposes of the present invention.

"Cancer stem cells", "tumor stem cells" or "tumor inducing cells", as defined herein, are cells with properties very similar to "normal" stem cells, exhibiting the most important property of self-renewal. Various cancers include cancer stem cells, rare cells with indefinite potential for self-renewal, that drive tumorigenesis. Cancer stem cells represent a small population of cells in tumors that resemble adult stem cells, the self-replicating cells that give raise to specialized cells in tissues. Like "normal" stem cells, cancer stem cells can seed the growth of new tissue, in this special case of new cancers, since it has been shown that implantation of a few tumor stem cells into mice led to the generation of all the cells of the original tumor; see for example Al-Hajj et al., Proc. Natl. Acad. Sci. USA published online February 24, 2003; Reya et al., Nature 414 (2001), 105-111; Miyamoto et al., Proc. Natl. Acad. Sci. USA 97 (2000), 7521-7526.

"Differentiation" is the process whereby relatively unspecialized cells (e.g., stem cells) acquire specialized structural and/or functional features characteristic of mature cells. Similarly, "differentiate" refers to this process. Typically, during differentiation, cellular structure alters and tissue-specific proteins appear.

"Feeder cells" or "feeders" are terms used to describe cells of one type that are co-cultured with cells of another type, to provide an environment in which the cells of the second type can grow. The feeder cells are optionally from a different species than the cells they are supporting. For example, certain types of ES cells can be supported by primary mouse embryonic fibroblasts, immortalized mouse embryonic fibroblasts (such as murine STO cells, e.g., Martin and Evans, Proc. Natl. Acad. Sci. USA 72 (1975), 1441-1445), or human fibroblast-like cells differentiated from human ES cells. The term "STO cell" refers to embryonic fibroblast mouse cells such as are commercially available and include those deposited as ATCC CRL 1503.

Most primary cultures, finite cell lines, and continuous cell lines are anchorage dependent and thus grow in monolayers attached to a surface. Other cells, particularly stem cells and tumor stem cells are anchorage independent and grow in suspension. Depending upon the cell type, suspension cultures are usually seeded at densities from 2 × 10⁴ to 1 × 10⁶ viable cells/ml and can attain densities of 2 × 10⁶ cells/ml. If cells are seeded at a too low density they will go through a lag phase of growth, grow very slowly, or die out completely. If cell densities are allowed to become too high, the cells may exhaust the nutrients in the medium and die abruptly. Recommended seeding and subculturing densities, media replenishment (feeding) schedules, and medium formulations for cell lines are provided on the product information sheet as well as in the catalog description on the Web sites of the ATCC or DSMZ. After the primary cell culture has bee established from, e.g., an embryonic stem cell line or a carcinoma tissue, subculturing of cell suspensions in accordance with the present invention may be performed with 1 × 10⁵ to 1 × 10⁶ viable cells/ml medium suspension; see also the examples. If necessary, the atmosphere of the cell culture container may be gazed with sterile-filtered CO₂, N₂ and/or a gas-controllable bioreactor may be used in order to keep the cell culture under hypoxic conditions. It is generally not necessary to completely change the medium unless the cells attain a very high density or the medium has an acidic pH (yellow in color from the phenol red). To completely replace the medium, the cells may be gently centrifuged (10 min at 125 to 250 × g), the medium decanted, and then the cells resuspened in fresh medium at a lower seeding density. Preferably, prior to centrifugation the suspension is transferred to another container in order remove non-stem cells which adhere to the culture surface, or which already died, for example because of the phenomenon called "anoikis".

As used herein, the term "substantially in suspension" refers to a plurality of stem cells being in suspension in a liquid culture with preferably the majority of stem cells being in suspension. The term "suspended" as used herein refers to those cells in a liquid media that are not adherent to the container holding the liquid media. The suspended cells may be considered as having as its majority continuously dividing cells.

In vitro, proliferating stem cells, in particular embryonic stem cells follow a reproducible temporal pattern of development that in many ways recapitulates early embryogenesis. Thus, as stem cells and their derivatives proliferate and differentiate, they typically form spherical tissue-like structures (spheroids), in some instances also called Embryoid Bodies (EBs). Other pluripotent stem cells such as neural stem cells have also been described to form spheroids. In the case of neural stem cells these spheroids are termed neurospheres. Unless stated otherwise the terms "spheroid" and "spheres" are used interchangeably herein and refer to a certain "ball-shaped" globular structure, consisting of more than a single cell that has initially developed from a single or from multiple cells. Accordingly, in specific embodiments of the present invention, the term "substantially in suspension" includes or refers to the formation of spheres or spheroids from stem cells. Though spheres and spheroids may not always be present in a suspended form, they can be detached from the container by gentle tapping or agitation of the culture, if necessary, contrary to for example true adherent non-stem cells. Furthermore, as demonstrated in the examples cells singled out of such "spheroid" and "spheres" give rise to such cellular structures anew, i.e. they behave like true stem cells.

The initial mixture of cells may be a group of cells or tissue fragments comprising multiple cells, although it is beneficial to have singular cells rather than tissue fragments. The continual passaging of the suspended cells may employ any suitable method, such as pipetting, pouring, or automated liquid transfer device for cell culture, and so forth, as long as it facilitates the transfer of suspended cells, including at least the majority of stem cells. In specific embodiments, the suspended cells may be centrifuged prior to delivery to a subsequent culturing container, which may comprise fresh media. Such centrifugation may occur upon one passage, upon more than one passage, or at every passage, but in specific embodiments it does not occur in at least the first passaging.

As used herein, the term "passaging" refers to the transfer of at least some cells in a first container having liquid media to a second container having liquid media. The transfer may include at least some media from the first container. The passaging may be to facilitate continued proliferation and to provide sufficient nutrients to the cells, such that an acceptable density threshold is not surpassed in a given container. Preferably, suspended cells of the present invention, either single cells or spheres/spheroids are transferred (passaged) into a fresh container more than once; most preferably they are successively passaged in liquid media in consecutive containers, wherein the passaging occurs at a frequency of about once every two to three days.

The culturing container may be of any suitable shape or material such that it distinguishes cells that adhere to each other and/or to substrates from cells that do not adhere to each other or to a substrate. In a particular embodiment of the present invention, the container comprises an additional substrate to which any kind of cell may adhere. In specific embodiments, the container shape is a conical, rectangular, spherical, or semi-circular flask or a tissue culture Petri dish, for example. In other embodiments, though less preferred, the container material is glass or plastic. In particular embodiments, the container material is untreated and comprises no specific agent placed thereon to facilitate adherence or non-adherence of the cells. In further specific embodiments, the container material is biologically inert. Most preferably, the surface of the culture container is coated with L-amino acids, especially poly-L-lysine and poly-L-ornithine.

The term "enrich" as used herein refers to increasing the quantity of non-differentiated cells, and in particular embodiments it refers to increasing the ratio of non-differentiated cells to differentiated cells. This may be further described as successively isolating stem cells from undesired differentiated cells. Usually, the ratio of stem cells to non-differentiated cells increases by a factor 2, preferably 5 and most preferably by a factor of more than 10. This may be even further defined as generating increasingly pure stem cell cultures with each successive passage. The enriched population of stem cells obtained in accordance with the method of the present invention is preferably substantially free of non-stem cells. After the suspended cells have been passaged one or more times, preferably at least about 10%, 20%, 30%, 40%, 50%, 60%, 70%, 80%, 90%, 95%, 97%, 99%, or 100% of the viable cells in the suspension are stem cells. Most preferably, the stem cells obtained in accordance with present invention are characterized by maintaining their pluripotency or multipotency; see supra.

The term "anoikis" refers to a sort of apoptosis of cells that have lost contact with extracellular matrix, or that interact with matrix through an inappropriate integrin-matrix combination. Anoikis characteristically occurs in epithelial, endothelial and muscle cells, as well as in oligodendrocytes, but also in fibroblasts subjected to growth factor deprivation, possibly by different mechanisms. Anoikis occurs through established apoptotic signaling pathways, which depend upon the cell type. These include caspases of both the initiator (caspase-8) and effector (caspase-3, 7) types as well as protein kinases such as MEKK-1/JNK. Protection against anoikis is afforded by the activation of certain other kinases such as FAK and Akt, whereas Bcl-2/Bcl-xL involvement depends upon cell type. In certain epithelial cell lines, cells must achieve complete cell-cell contact while attached to matrix, to become sensitive to anoikis. This sensitivity can be lost by activated oncogenes such as ras, alterations in intracellular apoptosis components, overexpression of growth factors such as EGF, HGF or IGF-related molecules, or breakdown of cadherin/catenin complexes. Thus, transformation by oncogenes or loss of tumor suppressor genes can render tumor cells resistant to anoikis, promoting anchorage independent growth/metastasis. Cells undergoing anoikis show typical apoptotic characteristics like for example, nucleosomal DNA ladder formation, cell shrinkage, caspase activation/cleavage of caspase substrates, as well as cytochrome c release from mitochondria. Apoptosis is triggered by several stimuli including hypoxia, radiation-induced DNA damage, oxidative stress and lack of attachment. In context with the present invention, the term "anoikis" preferably defines the type of apoptosis induced in proliferating and differentiated cells after proper adherence to the extracellular matrix (ECM) or to an equivalent substrate is disturbed or attachment to a substrate is prevented at all.

The terms "nucleic acid", "polynucleotide" and "nucleic acid sequence" refer to a polymer of nucleotides of any length. Included are genes and gene fragments, mRNA, tRNA, rRNA, ribozymes, cDNA, recombinant polynucleotides, branched polynucleotides, plasmids, vectors, isolated DNA and RNA, nucleic acid probes, and primers. As used in this disclosure, the term "polynucleotides" refer interchangeably to double- and single-stranded molecules. Unless otherwise specified or required, any embodiment of the invention that is a polynucleotide encompasses both a double-stranded form, and each of the two complementary single-stranded forms are known or predicted to make up the double-stranded form. As such, the nucleic acid sequence can, for example, include one or more exons, with or without, as appropriate, introns, as well as one or more suitable control sequences. In one example, the nucleic acid molecule contains a single open reading frame which encodes a desired nucleic acid product. The nucleic acid sequence is operably linked to a suitable promoter. A nucleic acid sequence encoding a desired nucleic acid product can be isolated from nature, modified from native sequences or manufactured de novo, as described in, for example, Ausubel et al., Current Protocols in Molecular Biology, John Wiley & Sons, New York (1998); and Sambrook et al., Molecular Cloning: A Laboratory Manual, 2nd edition, Cold Spring Harbor University Press, New York(1989). Nucleic acids can be isolated and fused together by methods known in the art, such as exploiting and manufacturing compatible cloning or restriction sites. Included are nucleic acid analogs such as phosporamidates and thiophosporamidates.

A cell is said to be "genetically altered", "transfected", "genetically transformed" or "transgenic" when a polynucleotide has been transferred into the cell by any suitable means of artificial manipulation or where the cell is a progeny of the originally altered cell that has inherited the polynucleotide. The polynucleotide will often comprise a transcribable sequence encoding a protein of interest, which enables the cell to express the protein at an elevated level. The term "transformed cells" includes stably transformed cells in which the inserted DNA is capable of replication either as an autonomously replicating plasmid or as part of the host chromosome, as well as transiently transformed cells which express the inserted DNA or RNA for limited periods of time. The genetic alteration is said to be "inheritable" if progeny of the altered cell have the same alteration.

In the context of encoding sequences, promoters, and other genetic elements, the term "heterologous" indicates that the element is derived from a genotypically distinct entity from that of the rest of the entity to which it is being compared. For example, a promoter or gene introduced by genetic engineering techniques into an animal of a different species is said to be a heterologous polynucleotide. An "endogenous" genetic element is an element that is in the same place in the chromosome where it occurs in nature, although other elements may be artificially introduced into a neighboring position.

The term "operably linked", as used herein, is defined to mean that the gene (or the nucleic acid sequence) is linked to control sequences in a manner which allows expression of the gene (or the nucleic acid sequence). General, operably linked means contiguous.

The terms "polypeptide", "peptide" and "protein" are used interchangeably in this disclosure to refer to polymers of amino acids of any length. The polymer may comprise modified amino acids, it may be linear or branched and it may be interrupted by non-amino acids.

As used herein, the term "microarray" refers to cell-based arrays, nucleic acid or nucleotide arrays or protein or peptide including antibody arrays that can be used to detect biomolecules, for instance to measure gene expression. "Array", "bioarray", "microarray", "nylon filter", "slide," and "chip" are used interchangeably in this disclosure. Various kinds of arrays are made in research and manufacturing facilities worldwide, some of which are commercially available. There are, for example, two main kinds of nucleic acid arrays that differ in the manner in which the nucleic acid materials are placed onto the array substrate: spotted arrays and in situ synthesized arrays. One of the most widely used oligonucleotide arrays is GeneChip^{™} made by Affymetrix, Inc. The oligonucleotide probes that are 20- or 25-base long are synthesized in silico on the array substrate. These arrays tend to achieve high densities (e.g., more than 40,000 genes per cm²). The spotted arrays, on the other hand, tend to have lower densities, but the probes, typically partial cDNA molecules, usually are much longer than 20- or 25-mers. Representative types of spotted cDNA arrays include LifeArray made by Incyte Genomics and DermArray made by IntegriDerm (or Invitrogen). Pre-synthesized and amplified cDNA sequences are attached to the substrate of these kinds of arrays. Protein and peptide arrays also are known; see, e.g., Zhu et al., Science 293 (2001), 2101. The term "microarray", as used herein, also includes high-density arrays of cells and tissues, in particular comprising stem cells for simultaneously examining complex biological interactions, which are identified by a specific location on a slide array. For example, a scanning microscope detects the bound, labeled sample and measures the visualized probe to ascertain the activity of the genes of interest in genotyping, cellular studies and expression analysis. It is a powerful technology that allows the simultaneous measurement of expression levels for up to tens of thousands of genes. Usually microarrays are used to simultaneously study large numbers of genes and their regulation, by probing with labeled nucleic acids taken from biological samples.

Microarray data, as used herein, encompasses any data generated using various arrays, including but not limited to the nucleic acid arrays described above. Typical microarray data include collections of gene expression levels measured using nucleic acid arrays on biological samples of different biological states and origins. The methods of the present invention may be employed to analyze any microarray data; irrespective of the particular nucleic acid microarray platform (e.g., nylon filters, glass slides, plastic, or silicon chips) from which the data are generated.

Gene expression, as used herein, refers in general to the transcription from DNA sequences into RNA molecules, which encode certain proteins with structural, enzymatic, or regulatory functions. The expression level of a given gene measured at the nucleotide level refers to the amount of RNA transcribed from the gene measured on a relevant or absolute quantitative scale, and in general refers to the relative abundance of the accumulated mRNA transcript. The expression level of a given gene measured at the protein level refers to the amount of protein translated from the transcribed RNA measured on a relevant or absolute quantitative scale. The measurement can be, for example, an optical density value of a fluorescent or radioactive signal, on a blot or a microarray image. Differential expression, as used herein, means that the expression levels of certain genes, as measured at the RNA or protein level, are different between biological samples in different states, tissues, or type of cells. Differential expression may also be observed relative to a reference standard. Such standard may be determined based on the context of the expression experiments, the biological properties of the genes under study, and/or statistical significance criteria.

Simple ratio, as used herein, refers to, with respect to a gene, is the ratio of its hybridization intensity measured from a first sample or a first group of samples to its hybridization intensity measured from a second sample or a second group of samples.

The first and second samples or groups of samples may be from different types of cells; or they may correlate with different biological and/or pathological states, according to various embodiments of this invention. The hybridization intensities may be normalized before the ratio is calculated according to certain embodiments, to account for the background noise, the bias introduced by the different samples, among other things.

The foregoing methods are preferably conducted at least twice on a given sample using at least one different primer pair specific for a specific marker gene. Following detection, one may compare the results seen in a given stem cell with a statistically significant reference group of reference stem cells. In this way, it is possible to correlate the number and kind of markers with various clinical states, toxicity values, or disease prognosis.

As used herein, "profile" or "profiling" of a cell, in particular stem cell or chemical composition or compound refers to a pattern of alterations in gene or protein expression, or both, or physiological properties in an ES cell, tumor cell, cancer stem cell, tissue, etc. contacted by the chemical composition compared to a like cell or tissue in contact only with culture medium.

If not stated otherwise the terms "compound", "substance" and "(chemical) composition" are used interchangeably herein and include but are not limited to therapeutic agents (or potential therapeutic agents), growth factors, lineage commitment factors, food additives and nutraceuticals, agents of known toxicities such as neurotoxins, hepatic toxins, toxins of hematopoietic cells, myotoxins, carcinogens, teratogens, or toxins to one or more reproductive organs. The chemical compositions can further be agricultural chemicals, such as pesticides, fungicides, nematicides, and fertilizers, cosmetics, including so-called "cosmeceuticals", industrial wastes or by-products, or environmental contaminants. They can also be animal therapeutics or potential animal therapeutics. Compounds to be screened may also be obtained from diversity libraries, such as random or combinatorial peptide or non-peptide libraries. Many libraries are known in the art that can be used, e.g., chemically synthesized libraries, recombinant (e.g., phage display libraries), and in vitro translation-based libraries.

### General Techniques

For further elaboration of general techniques useful in the practice of this invention, the practitioner can refer to standard textbooks and reviews in cell biology, tissue culture, embryology, and tumor biology.

With respect to tissue culture and embryonic stem cells, reference can be made to Teratocarcinomas and embryonic stem cells: A practical approach (Robertson, ed., IRL Press Ltd. 1987); Guide to Techniques in Mouse Development (Wasserman et al. eds. , Academic Press 1993); Embryonic Stem Cell Differentiation in Vitro (Wiles, Meth. Enzymol. 225 (1993), 900); Properties and uses of Embryonic Stem Cells: Prospects for Application to Human Biology and Gene Therapy (Rathjen et al., Reprod. Fertil. Dev. 10 (1998), 31).

General methods in molecular and cellular biochemistry can be found in such standard textbooks as Molecular Cloning: A Laboratory Manual, 3rd Ed. (Sambrook et al., Harbor Laboratory Press 2001); Short Protocols in Molecular Biology, 4th Ed. (Ausubel et al. eds., John Wiley & Sons 1999); Protein Methods (Bollag et al., John Wiley & Sons 1996); Non-viral Vectors for Gene Therapy (Wagner et al. eds., Academic Press 1999); Viral Vectors (Kaplitt & Loewy eds., Academic Press 1995); Immunology Methods Manual (Lefkovits ed., Academic Press 1997); and Cell and Tissue Culture: Laboratory Procedures in Biotechnology (Doyle & Griffiths, John Wiley & Sons 1998). Reagents, cloning vectors and kits for genetic manipulation referred to in this disclosure are available from commercial vendors such as BioRad, Stratagene, Invitrogen, Sigma-Aldrich, and ClonTech.

### Detailed description of the invention

The present invention relates to a cell culture system consisting of a serum-free medium and supplements as well as a support of culturing stem cells in a plurality of cells, which cell culture system exploits the adherence/anchorage dependency of differentiating and differentiated cells, as opposed to the non-adherence/anchorage dependency of stem cells in liquid media.

In one aspect, the present invention is based on the novel approach to look for stem cells and cancer stem cells in liquid suspension, since in normal cell cultures the non-adherent cells are removed with every change of medium. Since Friedenstein (Friedenstein, Exp. Hemato. 4 (1976), 267-274) postulated that mesenchymal stem cells adhere best to cell culture plastic, said mesenchymal and other stem cells are mainly selected according to this criterion, i.e. contrary to the here described invention. The suspension cells also exhibit stem cell character and are probably more potent. In some cases even differentiation is induced using for example dexamethason to achieve plastic adherence. In the cell culture system according to the present invention, the medium including the suspended cells is removed and separated from the cells by gravitation or centrifugation. The thereby obtained cells are resuspended in fresh medium and put back into the culture. As demonstrated in the examples, stem cells obtainable in accordance with the cell culture system of the present invention maintain their pluripotency and multipotency, respectively, and can be easily differentiated, for example into adipocytes, osteoblasts, neuronal cells, fibroblasts and smooth muscle cells. Furthermore, the cell culture system of the present invention could be shown to be applicable to nearly all kind of stem cells, e.g. human embryonic-like stem cells isolated from tissue (Example 1), human pluripotent, embryonic teratocarcinoma cell lines (Example 2), human mesenchymal stem cells (Example 3), stem cells derived from a human epithelial-like breast cell line (Example 4), stem cells from human breast carcinoma cell line (Example 5), and stem cells from ovarian carcinoma tissue (Example 6).

Furthermore, without intending to be bound by theory it is believed that the actual problem consists in the provision of an environment allowing the multipotent stem cells to be preserved in their early state of development and to proliferate like in a "niche" without being overgrown by the faster dividing transient amplifying (TA) cells and without being stimulated to differentiate. The thus processed cells should fulfill the criteria for their application in the human system and should be enriched. In this context the term "niche" is related to certain areas within the body, where stem cells can survive for a long time, since those areas provide an appropriate environment containing the "proper" signals (Spradling et al., Nature 414 (2001), 98-104).

Concerning cancer stem cells, it is important to observe the cells in a niche, enabling the cells to behave as in a native tumor. The significance of cytostatic tests in cell cultures is controversial and the correlation of the results compared to those of the clinical courses is not very high. Thus, improvement by a more significant cell culture system is desirable. Cancer stem cells are held responsible especially for the formation of multiresistance. With the present cell culture system said cancer stem cells can now, for the first time and with only little effort be selectively enriched, expanded and investigated; see Example 2, 5 and 6. A prerequisite for the selective enrichment of stem cells is that one or more characteristics can be found for the discrimination between stem cells and other cells, then enabling a selection.

The solution to these technical problems is provided by the combination of an appropriate serum-free medium containing supplements and an appropriate cell culture surface, exploiting the basic properties of stem cells for their selection and expansion. An additional advantage of the present invention is the controlled environment, in which the cells are transferred. By this means, cells can be processed for transplantation and for in vitro analyses.

Thus, the present invention provides a method for the enrichment of stem cells in a suspension of a plurality of cells, comprising culturing said plurality of cells in a first container comprising a culture medium under conditions allowing stem cells substantially in suspension and separating stem cells from non-proliferating cells by passaging the suspension into a second container, thereby enriching the stem cells.

The person skilled in the art will recognize that a suitable culture medium is used in the present invention such that stem cells may proliferate and preferably such that stem cells may be distinguished from non-stem cells, such as differentiated cells. Many suitable media are commercially available, such as from Invitrogen-Gibco (Carlsbad, California) or Sigma (St. Louis, MO), which can be adapted according the teaching provided herein. The media utilized may be serum-free or serum-comprising, although serum-free medium is preferred to avoid cell exposure to one or more pathogens. As already mentioned, one advantage of the cell culture system of the present invention is the lack of need of the step of co-cultivation with feeder cells. Preferably, the culture medium of the present invention is substantially serum-free and substantially a non-conditioned medium. Accordingly, the culture medium of the present invention usually does not comprise serum and lacks feeder cells, matrix or both.

In one particularly preferred embodiment of the present invention, the culture medium comprises Knockout-Dulbecco's modified Eagle's medium (KO-DMEM). Appropriate serum-free media such as KO-DMEM as well as culture medium supplements can be obtained by commercial suppliers, for example Invitrogen; see also supra. Furthermore, components of the cell culture media of the present invention such as KO-DMEM and KO Serum Replacement as well as other ingredients, e.g. vitamins, antioxidants, and more trace elements are described in international application WO98/30679, the disclosure content of which is incorporated herein by reference. In this context, the person skilled in the art will recognize that the applications of the cell culture medium described in WO98/30679 such as methods of producing transgenic animals and recombinant proteins from such transgenic animal can be applied to the cell culture system of the present invention as well. Accordingly, embodiments described for the use of cell culture medium in international application WO98/30679 are included in the scope of the present invention with the proviso for some embodiments that a cell culture medium as disclosed and claimed herein is used. The same naturally applies to embodiments that relate to the use of a cell culture container of the present invention. Furthermore, reference is made to international application WO2005/046596, in particular at section III, paragraphs [0074] to [0080] describing several components of culture media that can be utilized for preparing a cell culture medium in accordance with the present invention.

DMEM Knockout (Invitrogen) is a medium that has been optimized for ES cells and is optimally functioning for the purpose of the present invention. Additionally advantageous is also the use of calcium-reduced or calcium-free medium, providing an additional contribution to the selection of stem cells, since thereby somatic cells like for example fibroblasts in contrast to the stem cells are inhibited regarding their growth, and additionally a lot of calcium-dependent proteins cannot be activated. Knockout SR (Invitrogen) is a serum substitute with an exactly defined protein composition not containing any growth factors, hormones or other imponderabilities. Further serum substitutes can also be provided by the use of defined soya protein extracts, yeast extracts or other defined protein extracts.

ES cells cultivated under serum-free conditions, produce FGF-4, which inhibits endodermal and mesodermal differentiation. This enables the stem cells to differentiate mainly ectodermal. Thus, in one embodiment of the method of the present invention, the culture medium preferably comprises Fibroblast Growth Factor-4 (FGF-4).

Said ectodermal differentiation is inhibited by the addition of TGF-β and BMP4, respectively. TGF-β2 additionally activates NFκB, which leads to an increase of the viability of tumor stem cells, as well as to the inhibition of apoptosis in tumor cells (Lu, Proc. Natl. Am. Soc. 101 (2004), 7112-7117). Thus, in a further embodiment of the present invention, the culture medium additionally or alternatively comprises Tumor Growth Factor-beta (TGF-β). TGF-β is produced by fibroblasts (part of the feeder effect) and also plays an important role in the modeling of the oncogenic potential of vicinal epithelial cells (Bhowmick, Science 303 (2004), 848-851). The effect of TGF-β is mediated by the receptors TGF-β receptor types I and II (RI and RII). Binding of the ligands to TGF-β RII leads to its phosphorylation, and as a consequence to the phosphorylation of TGF-β RI, which then can also bind TGF-β. This results in different intracellular signal transductions via the MAPK cascade and the transcription factors API and SP1. TGF-β RI also activates signals by phosphorylation of SMAD proteins, those becoming effective in the nucleus, a connection which has been described for epithelial cells (Attisano, Science 296 (2002), 1646). Of special interest is also the antiproliferative effect of TGF-β on epithelial cells.

In a still further embodiment of the present invention, the culture medium comprises Bone Morphogenetic Protein-4 (BMP-4.) BMP-4 supports the undifferentiated growth of embryonic stem cells by inducing extracellular receptor kinase (ERK) and p38 mitogen-activated protein kinase (MAPK). The division of the cells can be stimulated by further addition of mitogenic growth factors like for example bFGF, EGF or PDGF-BB; see also infra.

An accelerated differentiation of precursor cells or transient amplifying (TA) cells can be stimulated to by providing culturing conditions preventing only a few and a few but specific, respectively, mitotic signals, taking advantage of the property that an elongated cell cycle leads to differentiation. In contrast to stem cells, differentiated cells in most cases need different mitogens in higher amounts. By this, stem cells can be expanded selectively and only those are able to survive in the cell culture medium for a long time. Therefore, according to the invention the method comprises the cultivation of the plurality of cells under conditions inducing differentiation of precursor cells and/or transient amplifying (TA) cells in the presence of a mitotic agent, preferably a mitogenic growth factor selected from the group consisting of basic Fibroblast Growth Factor (bFGF), Epidermal Growth Factor (EGF), Platelet Derived Growth Factor-BB (PDGF-BB), Insulin-Like Growth Factor (IGF), Vascular Endothelial Growth Factor (VEGF), Stem Cell Factor (SCF), Interleukin-2 (IL-2), IL-6, TGF-β1 and TGF-β2. Most preferably, at least bFGF is present in the culture medium; see also the examples.

Nucleotides originate by the attachment of a phosphate to the sugar moiety of the nucleosides and have a lot of functions within the cells as for example carriers of energy, in the formation of coenzymes or as specific signal molecules (cAMP). Thus, in one embodiment of the present invention, the culture medium comprises nucleosides, preferably in the amount of 0.1 to 5%. The above described addition of mitogenic factors and/or the addition of the nucleosides adenosine, guanosine, thymidine, cytosine and uridine result in accelerated G1 and S phases of the stem cells, which mainly leads to symmetrical division. According to the present invention, a self-produced nucleoside stock solution (adenosine 3 µmol/ml, guanosine 3 µmol/ml, thymidine 1 µmol/ml, cytosine 6 µmol/ml and uridine 3 µmol/ml) is preferably used; see also the examples.

In the examples, 2-mercaptoethanol is added to the culture medium as reducing factor. Thus, in one embodiment of the present invention, the culture medium comprises a reducing agent. Appropriate reducing agents include but are not limited to 2-mercaptoethanol, monothioglycerol, and dithiothreitol. Most preferably, 2-mercaptoethanol is used.

As a further additive, non-essential amino acids release the metabolism of the stem cells, since they need not be synthesized by the cells. This also leads to an accelerated course of the cell cycle. Thus, in one further embodiment of the present invention, the culture medium comprises non-essential amino acids.

The agile metabolism of the cells leads to a fast decrease of the pH value of the medium, detectible by the yellowing of the pH indicator. Especially outside of the incubator, the pH value can change fast and negatively influence the cells. This process can be retarded by the addition of organic buffer substances like for example HEPES. Thus, since conditions for culturing should be close to physiological conditions, meaning that the pH of the culture medium should be close to physiological pH, in one further embodiment of the present invention, the culture medium comprises a buffer for maintaining the pH at between pH 6-8, more preferably between about pH 7 to 7.8, with pH 7.4 being most preferred. In this context, a pH indicator such as Phenol red may be added to the culture medium. At low pH levels, phenol red turns the medium yellow, while at higher pH levels it turns the medium violet. For most tissue culture work (pH 7.4), the medium is bright red.

To culture the cells as close as possible to physiological conditions, the temperature should also be physiological, i.e. in a range between about 30°C to 40°C. Thus, cells are preferably cultured at temperatures between about 32°C to about 38°C, and more preferably between about 35°C to about 37°C.

L-glutamine or stable glutamine is preferably also added to the medium and used by the cells for their metabolism, thereby being catabolized either without enzymes into pyrrolidon carbonic acid and ammonia or via glutaminase into glutamic acid and ammonia. Accordingly, in a further preferred embodiment of the methods of the present invention, the culture medium comprises L-glutamine or stable glutamine or any other equivalent nitrogen providing compound which can be metabolized by the cell.

In case of contamination with bacteria or fungi, additionally antibiotics like for example penicillin/streptomycin, gentamycin, or antimycotics like for example Amphotericin B or nystatin are added to the culture medium according to standard cell culture protocols; see also Examples 1 and 6. The addition of these substances increase the osmolarity of the medium which may provide a further advantageous selection for stem cells since they appear to tolerate higher osmolarity better than non-stem cells.

Furthermore, as mentioned above, it could be observed that normal tissue cells do not proliferate under the present culture conditions and that TA cells rapidly complete differentiation. Indeed, an enrichment of vital stem cells and thereby the formation of spheres could be observed. In particular, the latter effect could be enhanced by the presence of E-cadherin in the culture medium, an agonist of the Wnt-pathway. Thus, in one preferred embodiment of the present invention, the culture medium comprises a Wnt-pathway activating or upregulating substance. This may be, e.g., a soluble Wnt3a factor or a functional part, derivative or analog thereof. The Wnt signalling pathway has been reviewed by Prunier et al., Growth Factors 22 (2004), 141-150, which also describes agonists and antagonists of the Wnt signal transduction pathway. Furthermore, a small molecule agonist of the Wnt signalling pathway has been described by Liu et al., Angew. Chem. Int. Ed. Engl. 44 (2005), 1987-1990. Most preferably, said Wnt-pathway activating substance comprises E-cadherin or a cadherin agonists, for example internalin.

As already mentioned above, stem cells and cancer stem cells seem to be more stable than non-stem cells, since they can protect themselves and therefore in accordance with the present invention should tolerate higher osmolarities than differentiated cells, which also contributes to selection. Thus, in one embodiment of the present invention, the osmolarity of the culture medium is increased in relation to a basic culture medium, e.g. the osmolarity of said culture medium may be higher than about 350 mOsmol.

The selection of the growth factors together with the effects of the single added components as well as the choice of the medium leads in the present invention to the selection of stem cells and cancer stem cells, respectively, as well as to the development of their properties. In this context, it has already been explained hereinbefore that the present invention exploits the anchorage dependency of non-stem cells on the one hand, and the mobility of stem cells and the appropriate choice of the surface of the culture surface on the other hand.

Accordingly, in one embodiment the method of the present invention comprises a step of culturing the cells under conditions preventing adherence of stem cells and non-proliferating cells to the surface of the culture container. In particular, the method of the present invention comprises the cultivation of a plurality of cells under conditions for the selective induction of apoptosis (anoikis) in non-proliferating cells, by exploiting the adherence-dependency of non-proliferating cells and the non-adherence-dependency of stem cells, respectively. Separation is herein achieved by an apoptotic process (anoikis) resulting from the detachment of the cells from an extracellular matrix, whereafter only suspension cells (which are not dependent on adhesion) or cells able to produce their own extracellular matrix (ECM) survive in suspension, whereas adherence-dependent cells which do not have said properties undergo apoptosis.

Adherence of cells to a substrate can be blocked by various means, for example blocking substances selected from the group consisting of human serum albumin, animal serum albumin, gelatin, Ficoll 70 and Ficoll 400, preferably by hydrogel, teflon or nanoparticles. In one embodiment of the present invention, the surface of the container is coated with the blocking substance.

International application WO2005/046596 relates to methods of enriching stem cells in a mixture of stem cells and non-stem cells aiming at exploiting the non-adherent property of stem cells, as opposed to the adherent property of differentiating the cells. In this context, the invention claimed in WO2005/046596 depends on the presence of a substrate to which non-proliferating cells substantially adhere, while stem cells do not. Thus, according to the teaching of this international application, a substrate has to be provided to which specifically non-proliferating cells adhere, and which usually would cover the surface of the container comprising the liquid cell culture medium. In contrast, the present invention does not have to rely on a substrate or surface which is selective for non-differentiating cells. At least in some specific embodiments, the method of the present invention does not involve a culture container comprising a surface or substrate where only non-stem cells specifically and substantially adhere to. Accordingly, in at least some if not all embodiments of the present invention, a method for enriching stem cells in a plurality of cells as characterized in the claims of international application WO2005/046596 and specifically described in its examples is excluded from the scope of the present invention. However, for some embodiments of the present invention, any one of the methods described in WO2005/046596 may be incorporated, for example for the enrichment and isolation of tumor stem cells.

International application WO03/004626 relates to a method of generating cells from spheroid forming cells comprising culturing spheroid forming cells under controlled cell aggregation conditions that enable spheroid formation in liquid suspension. This international application requires the step of encapsulation of single cells, in particular embryonic stem cells so as to control cell aggregation such that each capsule will transiently contain and give rise to one embryoid body; see the examples of WO03/004626. As will be apparent from the description of the present application and the examples, the present invention does not require such measures. Accordingly, in most if not all embodiments, methods of generating embryonic stem cells as claimed in WO03/004626 and specifically described in the examples of this international application are excluded from the scope of the present invention. Nevertheless, in so far the methods described in WO03/004626 can be adapted to the methods of the present invention, they are of course encompassed herein by reference, in particular in so far the enrichment and isolation of non-embryonic stem cells, for example tumor stem cells, is concerned.

Stem cells originating from various types of tissue show common properties as well as common markers like for example Oct-4, which can only be found in pluripotent cells. A further common property is the increased mobility of the stem cells and cancer stem cells, respectively. Processes meant to induce repair of a damaged tissue in stem cells, cause metastazation of cancer stem cells. Cancer stem cells migrate through the whole body and attach to another site, where they differentiate according to the signals of their surrounding area and can grow into different metastases with various cell types, which effect has been observed upon comparison of different metastases. In accordance with the present invention, said common property is exploited to accelerate the selection of stem cells and cancer stem cells using an appropriate surface coating. Stem cells and cancer stem cells are supported by enzymes to pave their way through the environmental tissue.

Thus, in a further alternative embodiment, the method of the present invention comprises culturing the cells under conditions allowing the detachment of the cells, preferably substantially of stem cells only. Taking advantage of different enzymatic properties of stem cells and non-stem cells, in a preferred embodiment of the method of the invention said polypeptide is an enzyme, preferably an enzyme being capable of cleaving the substrate, more preferably being a protease and most preferably being a metalloprotease.

In a preferred embodiment of the present invention, the surface of at least one container, i.e. the surface of the culture container is coated with a substrate, such as a cell or tissue matrix protein or parts thereof to be cleaved by a protease specifically expressed by stem cells at an enhanced level compared to non-proliferating cells. Advantageously, stem cells, in contrast to "normal" cells, produce an increasing amount of proteases like for example matrix metalloproteinases. For example, it is believed that the release of gelatinase B (MMP-9), which is involved in the degradation of extracellular matrix molecules, might induce stem cell mobilization by cleaving matrix molecules to which stem cells are attached. In this context, agents such as MIP-1alpha and IL-8 may be added to the culture medium in order to stimulate the secretion of MMP-9 and probably other matrix metalloproteinases, thus facilitating detachment of the stem cells from the culture container's surface.

By choosing an appropriate adhesive coating like for example poly-L-lysine, poly-L-ornithine or other poly-L-amino acids, the stem cells are enabled to adhere to the surface of the cell culture container, but contrarily to differentiated tissue cells, they also are able to faster detach from the adhesive coating due to their increased protease activity. Due to the fact that the enzymes can affect L-forms of the amino acids like for example poly-L-lysine, but not the D-forms of amino acids like for example poly-D-lysine, poly-L-lysine is preferably used in accordance with the present invention as an effective coating for selection. Other coatings, enabling a fast enzymatical detachment via proteases are also appropriate for this kind of selection. Hitherto, the use of the above-described properties for the selection of stem cells has not been described. The especially interesting stem cells and cancer stem cells are mainly in suspension and grow mainly in spheroidal form due to bad adhesion to the cell culture container or another substrate, the spheres thereby adhering at the beginning, but then rounding and finally detaching themselves. An additional criterion for the selection is the adherence-dependency of tissue cells. Said cells undergo apoptosis when they cannot attach themselves. Thus, vital stem cells and cancer stem cells, respectively, are enriched in this supernatant.

Hence, according to the present invention, the composition of the components of the medium are preferably selected such that especially the discharge of the metabolism and the accelerated course of the cell cycle is taken into account, as well as the choice of cytokines inhibiting differentiation and their mitogenic and protective properties, in connection with a surface from which the cells can enzymatically detach and pass over in suspension. For this purpose, however, also surfaces can be used which enable a soft adherence of the cells like for example plastic ware treated for cell cultures (polystyrene), glass and CellBIND (Corning). Here the effect of detachment exists as well, but in contrast takes longer and is less effective.

The coatings described in the literature usually have the opposite purpose, namely to effectively "stick" the cells to the container or another substrate. Therefore, for these purposes coatings like poly-D-lysine, collagen, fibronectin, laminin, matrigel and others are used in stem cell technology. The cells are only rarely able to detach from these adhesion-mediative surface coatings. Thus, almost all cells adhere to the surface and the selection underlying the present invention cannot take place. Additionally, those materials provide strong stimulations for differentiation hampering the expansion of stem cells.

In certain embodiments, the method of the present invention comprises isolating cells of a heterogeneous population of cells which may be derived from tissue or culture, for example surgically isolated or taken from a heterogeneous cell culture and preferably, wherein the plurality of cells is obtained from dental follicle, a breast cell line, a carcinoma cell line, a breast cancer cell line, ovarian carcinoma, more preferably from bone marrow, blood, cord blood, umbilical cord, placenta, amniotic liquor, fat, organs like breast, ovary, skin, heart, liver, kidney, pancreas and malignant tumors of every origin including blood, brain, breast, ovary, prostate, lung and skin.

For at least the primary cell culture, the cell culture container's surface is preferably coated with L-amino acids, especially poly-L-lysine and poly-L-ornithine, since almost all cell types adhere to these basic amino acids and since additionally the L-forms of the amino acids are enzymatically degradable. CellBIND (Corning), glass surfaces as well as cell culture plastic are less effective but work as well. Poly-L-lysine (PLL) and poly-L-ornithine (PLO) are synthetic compounds that enhance cell adhesion and protein absorption by altering surface charges on the culture substrate. PLL and PLO do not stimulate biological activity in the cells cultured on them and they do not introduce impurities carried by natural polymers. Poly-L-lysine and poly-L-ornithine cellware such as vessels, multiwell and assay plates are commercially available, for example from BD Biosciences, Bedford, MA USA.

Furthermore, in accordance with the present invention, it was surprisingly found that in particular unselected adult primary cells and cell lines after seeding mainly adhered to the coatings of the cell culture container, but that then a reduction of the number of adherent cells could be observed, leading to the formation of three-dimensional globular "spheres" or "spheroids" still exhibiting growth, whereas the adherent cells did not continue to proliferate; see the examples. Furthermore, it was notable that on the ground beneath the spheres, cells with a fibroblastoidal morphology are attached, from which the spheres initially originate and to which they attach, respectively.

While growing, the spheres can be removed from the surface of the cell culture container by soft tapping or they detach by themselves only by the movement of the medium. The remaining cells often form new spheres. The spheres can be dissected into single cells by enzymatic digestion e.g. using a trypsin solution, a mixture of collagenase and dispase or Accumax (PAA) as well as by trituation with EDTA/DNAse, and afterwards be seeded into the same system. In the subcultures obtained thereby the formation of new spheres in big amounts was observable. This enables the simplified "handling" of the cells, which can be thereby selected and enriched, whereas the differentiated cells remain adhered in the previous culture flask. It was possible to observe that for a long time spheres even from remaining stem cells in the adherent fraction could have been regained, this recoupment decreasing with time. During the change of the medium it is especially important to back transfer the detached cells or cell aggregates into the system.

Especially surprising was the behavior of the cancer cell lines in the cell culture system of the present invention. Within a very short time they split up into adherent cells, suspension cells and spheres, wherein the suspension cells and spheres possess stem cell properties. This enables the selection and purification of cancer stem cells. Especially noticeable was the formation of cells having more than one nucleus, which could be observed for example in the breast cancer cell line MCF-7 (Example 5), a phenomenon, which to date was only found in native tumors. Thus, for the first time, an identical behavior could be shown in cell cultures.

In view of the above observations, in one embodiment, the method of the present invention comprises culturing the cells under conditions allowing the formation of spheres or spheroids of the stem cells, optionally further comprising gentle agitation of the cell culture in order to detach the spheres or spheroids from the culture container's surface, and selecting the spheres or spheroids thereby obtained.

Spheres are also used in embryonic stem cell technology, so-called "embryoid bodies", which in contrast to the present invention are not self-growing, but rather originate from the application of non-adherent culture plates (bacteriological plates or Ultra low Binding (Corning)), since the only possibility of the cells to adhere is to adhere to each other; accordingly, they have to form spheres. In ES cells, by this behavior, differentiation is stimulated. When spheres or single cells obtained from the spheres are posed on surfaces enabling an enhanced adherence of the cells like for example gelatin, laminin, matrigel or fibronectin, the cells exhibit a unique morphology. Concerning the different cell types of the primary culture, a selection is demonstrated.

The same medium leads to morphological amendments, differentiation after several passage and in some cases to death of the cells after a few passages, when cells are cultivated on gelatin, laminin, matrigel or fibronectin, whereas at the same time the sphere culture in the system as described above still produced spheres. The growth of the cells could be maintained for a time period of eight months (dental sack). In contrast to the monolayer cultures, the growth of the spheres was at a slower rate.

Hence, in one embodiment, the method of the present invention is preferably carried out in that the cells are cultivated under conditions allowing spheroid formation in liquid suspension, wherein a culture container with a non-adhering coating like hydrogel is used to prevent adhesion of the cells to the container; or wherein in the first step cells are seeded on matrices like gelatine to form colonies and in the second step the colonies are brought into such a container.

According to the present invention, another method for the modulation of proliferation and/or differentiation is hypoxicity. Without intending to be bound by theory, it is believed in accordance with the present invention that a given population of cells including stem cells is distributed along an oxygen (O₂) gradient, where stem cells reside in the most hypoxic areas and differentiated and differentiating cells are found in O₂-rich areas. Therefore, according to one preferred embodiment of the present invention, culturing of the cells is performed under O₂ levels below 20% O₂ (normoxia), for example between 19% O₂ and 0.5% O₂ (hypoxia), preferably at about 10% , 7% or 1.5% O₂ for a culture period of about 2 to 4 days, which however my be repeated one or more times. Most preferably, the O₂ level corresponds to the corresponding level in the tissue the stem cells have been isolated from. "Hypoxic" chambers are commercially available, for example from Coy Laboratory Products, Grass Lake, MI, USA.

Thus, in some embodiments of the invention, the cells are cultivated under hypoxic conditions, provided by reducing the oxygen concentration and/or the formation of spheroids. The hypoxic environment resembles the true situation of for example a cancer cell within a tumor, but also the situation of a "normal", healthy cell in a healthy body, as the oxygen concentration, either in a tumor tissue or in healthy tissue is lower than in an incubator traditionally used for cell culturing. According to the present invention, the hypoxic conditions can either be provided by an incubator which is aeratable with nitrogen or by a controlled bioreactor.

As demonstrated by the appended examples, the stem cells, cancer stem cells and the suspensions derived therefrom, useful in the context of the present invention, can be derived from a variety of sources including, but not limited to bone marrow, mobilized or immobilized peripheral blood, umbilical cord blood, fetal liver tissue, other organ tissue, skin, nerve tissue, solid tumors, etc. A variety of stem/progenitor cell types may advantageously be isolated and enriched according to the invention including, but not limited to lympho-hematopoietic stem cells, multipotent or pluripotent stem cells, mesenchymal stem cells, epithelial stem cells, gut stem cells, skin stem cells, neural stem cells, liver progenitor cells, endocrine progenitor cells, as well as cancer stem cells of various types of cancer.

Stem cells have been identified in a variety of tissues. They can be distinguished by a variety of means, such as by the tissue from which they were harvested, their bias in differentiation ability, the stage of development at which they exist, and their gene expression profile. In particular, stem cells may originate from ectoderm (epidermal, neural, neural crest, and hair follicle), mesoderm (cardiac muscle, skeletal muscle, umbilical cord blood, mesenchymal, hematopoietic, umbilical cord matrix, and multipotent adult precursor), endoderm (pancreatic islet and hepatic oval); or may be (primordial germ) stem cells. More than one stem cell may be present in a particular tissue. For example, in the hematopoietic system alone, there are stem cells from the yolk sac, fetal cord blood, liver and adult bone marrow.

The invention can be practiced using tissue or primary cells of any vertebrate species. Included are cells from human, as well as non-human primates, domestic animals, livestock, and other non-human mammals, in particular rodents such as mice and rat. Amongst the stem cells suitable for use in this invention are primate pluripotent stem cells derived from tissue formed after gestation, such as blastocysts, or fetal or embryonic tissue taken any time during gestation. Non-limiting examples are primary cultures or established lines of embryonic stem cells. The invention is also applicable to adult stem cells. It is referred to the literature of Anderson et al., Nat. Med. 7 (2001), 393-395; Gage, Science 287 (2000), 433-438 and Prokop, Science 276 (1997), 71-74.

In one embodiment of the present invention, said stem cells are multipotent or pluripotent stem cells. For some embodiments, said stem cells may be non-embryonic stem cells, embryonic carcinoma (EC) stem cells, mammalian embryonic stem (ES) cells, fetal stem cells, adult stem cells or tumor stem cells.

A surprising observation that has been made in accordance with the present invention, is that the culturing conditions for embryonic stem cells are also the "appropriate" conditions for adult stem cells as well as cancer stem cells to provide a niche in which they can proliferate without differentiating. For this reason, embryonic stem cell technology was applied to adult stem cells. Thus, in one aspect, the present invention relates to a composition comprising non-embryonic stem cells in a culture medium which may be substantially identical to any one of the serum-free, eukaryotic cell culture media described in international application WO98/30679. In particular, it has been surprisingly found that the cell culture media solely described in this international application for culturing and isolating embryonic stem cells, could also be adapted and used for the same purposes in respect to non-embryonic stem cells, in particular adult stem cells and tumor stem cells. This is particular surprising, since previously it has been thought that every type of stem cells deserves a unique medium composition for supporting the growth of the respective stem cells in culture. As demonstrated in the examples, this is not necessarily so. Advantageously, according to the methods of the present invention, one major cell culture medium can be used for culturing and isolating stem cells irrespective their origin. This finding of course substantially reduces the costs for research and development, since it is no longer necessary to prepare several different culture media but one.

Cancer stem cells like "normal" stem cells undergo "self-renewal" and "differentiation" to form a tumor, they give rise to abnormal cell types, and may change over time as additional mutations occur. The developmental origin of cancer stem cells can vary between different types of cancers. Cancer stem cells may arise either as a result of genetic damage that deregulates the proliferation and differentiation of normal stem cells (Lapidot et al., Nature 367 (1994), 645-648), or by the deregulated proliferation of a normal restricted progenitor or a normal differentiated cell type. Typically, tumors are visualized and initially identified according to their locations, not by their developmental origin.

By this invention, the principles of normal stem cell biology also have been applied to the isolation and characterization of cancer stem cells; see Examples 2, 5 and 6. Examples of tumors from which cancer stem cells can be isolated or enriched according to the invention include sarcomas and carcinomas such as, but not limited to: fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinoma, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.

Once stem cells are isolated, enriched and stably cultivated in accordance with the methods of the present invention, cell lines of said stem cells may be established according to methods known in the art. Hence, the present invention also relates to the populations of stem cells and spheroids obtainable by the method of the present invention. Most preferably, the stem cell or spheroid population of the present invention is substantially free of exogenous/heterologous cell components such as those are derived from fibroblasts or serum. Since it is known that flow cytometry-sorted stem cells are a mixed population with contaminating cells, the present invention avoids the contamination of non-stem cells by providing a substantially pure culture of stem cells. This may be defined as having a 100 % homogeneous population of stem/progenitor cells with no contaminating cells. The stem cell progeny obtained by the method of the present invention may be cryopreserved until they are needed by any method known in the art. The cells may be suspended in an isotonic solution, preferably a cell culture medium, containing a particular cryopreservant. Such cryopreservants include for example dimethylsulfoxide (DMSO), glycerol and the like and cells are frozen gradually to a temperature of approximately -150 °C.
Of course, it is envisaged by the present invention to administer or graft a stem cell of the present invention or derivatives thereof to a non-human animal, preferably mice, in order to investigate for example their tumorigenic potential.

In one embodiment of the method of the present invention, said stem cells are genetically engineered, i.e. they are transgenic stem cells. This embodiment is particularly suited if the stem cells are intended to be exposed to in vitro differentiation in order to deplete populations of differentiated cells of relatively undifferentiated cells and/or of cells of undesired cell types by using a selection system that is lethal to the undesired cells and cell types, i.e. by expressing a selectable marker gene that renders cells of a specific cell type resistant to a lethal effect of an external agent, under control of a regulatory sequence that causes the gene to be preferentially expressed in the desired cell type and/or at a certain stage of development. To accomplish this, prior to the process used to differentiate the cells into the desired lineage for therapy, the cells are genetically altered, in a way that the cells comprise a selectable marker and/or reporter gene operably linked to a cell type-specific regulatory sequence specific for the desired cell type.

Of course, the stem cells used in accordance with the present invention may also be transgenic because of other reasons, for example they express a therapeutically active protein and/or they have been genetically altered in order to suppress an inherited disease. Thus, a potential advantage of stem cells in addition to cell replacement therapy is that they can be genetically engineered in vitro to produce beneficial proteins. Those engineered stem cells may also be employed to deliver gene mouse models of various diseases like for example cancer diseases or neurodegenerative diseases. The effect of said engineered cells used in gene therapy and "produced" as described hereinbefore can be due to (over)expression of a (mutated) gene, knock-down of gene (e.g. by RNA interference; RNAi), knock-out, or knock-in of a gene.

Any suitable expression vector for the purposes of the present invention can be used. Suitable viral vector systems for producing stem cells altered according to this invention can be prepared using commercially available virus components. The introduction of the vector construct or constructs into the embryonic stem cells occurs in a known manner, e.g. by transfection, or with the help of viral vectors. Viral vectors comprising effector genes are generally described in the publications referenced to in the last section. Alternatively, vector plasmids can be introduced into cells by electroporation, or using lipid/DNA complexes. Exemplary is the formulation Lipofectamine 2000(TM), available from Gibco/Life Technologies. Another exemplary reagent is FuGENE(TM) 6 Transfection Reagent, a blend of lipids in non-liposomal form and other compounds in 80 % ethanol, obtainable from Roche Diagnostics Corporation. Furthermore, nucleofection of human embryonic stem cells has recently been reported by Nix et al., Stem Cells and Development 14 (2005), 378-383.

Resistance genes per se are known. Examples for these are nucleoside and aminoglycoside-antibiotic-resistance genes for, e.g. puromycin, streptomycin, bleomycin, neomycin, gentamycin or hygromycin. Further examples for resistance genes are dehydrofolate-reductase, which confers a resistance against aminopterine and methotrexate, as well as multi drug resistance genes, which confer a resistance against a number of antibiotics, e.g. against vinblastin, doxorubicin and actinomycin D.

It may be desirable that the cells have the ability to replicate in certain drug screening and therapeutic applications, and to provide a reservoir for the generation of in vitro differentiated cells such as neuronal cells and their precursors. The cells of this invention can optionally be telomerized to increase their replication potential, either before or after they progress to restricted developmental lineage cells or terminally differentiated cells. ES cells that are telomerized may be taken down the differentiation pathway described earlier; or differentiated cells can be telomerized directly.

Cells are telomerized by genetically altering them by transfection or transduction with a suitable vector, homologous recombination, or other appropriate techniques, so that they express the telomerase catalytic component (TERT), typically under a heterologous promoter that increases telomerase expression beyond what occurs under the endogenous promoter. Particularly suitable is the catalytic component of human telomerase (hTERT), provided in international application WO98/14592. For certain applications, species homologs like mouse TERT can also be used; see international application WO99/27113. Transfection and expression of telomerase in human cells is described in Bodnar et al., Science 279 (1998), 349, and Jiang et al., Nat. Genet. 21 (1999), 111. In another example, hTERT clones are used as a source of hTERT encoding sequence, and spliced into an EcoRl site of a PBBS212 vector under control of the MPSV promoter, or into the EcoRl site of commercially available pBABE retrovirus vector, under control of the LTR promoter; see also international application WO98/14592. They can then be assessed for hTERT expression by RT-PCR, telomerase activity (TRAP assay), immunocytochemical staining for hTERT, or replicative capacity; see also supra.

Continuously replicating colonies will be enriched by further culturing under conditions that support proliferation and cells with desirable phenotypes can optionally be cloned by limiting dilution. Depending on the intended use of the cells, other methods of immortalization may also be acceptable, such as transforming the cells with DNA encoding myc, the SV40 large T antigen, or MOT-2; see for example US patent No. 5,869,243 and international applications WO97/32972 and WO01/23555.

In another embodiment of the invention, one or more stem cells or cell lines thereof are differentiated by contacting a differentiation agent, and a composition is prepared comprising differentiated cells; see also the examples. After the stem cells have been propagated continuously in culture, differentiation can be promoted into a wide variety of cell types, including cells found in each of the endoderm, mesoderm, and ectoderm germ layers; see also the examples. With the appropriate combinations of growth and differentiation factors, however, cell differentiation can be controlled. Hence, the stem cells of the present invention can be differentiated in vitro and in vivo in to various cell types and progenitors thereof, including but not limited to hematopoietic cells, neuronal cells, pancreatic cells, hepatocyte precursor cells, adipocytes, chondrocytes, osteoblasts, retinal pigment epithelial cells, fibroblasts, skin cells such as keratinocytes, dendritic cells, hair follicle cells, renal duct epithelial cells, smooth and skeletal muscle cells, testicular progenitors, and vascular endothelial cells. Embryonic stem cell differentiation models for cardiogenesis, myogenesis, neurogenesis, epithelial and vascular smooth muscle cell differentiation in vitro have been generally described in Guan et al., Cytotechnology 30 (1999), 211-226. In vitro differentiated cardiomyocytes, neural cells, hepatocytes, adipocytes, skeletal muscle cells, vascular endothelial cells and osteoblasts are also described in US patent application US2002/142457.

In certain embodiments of the present invention, differentiation is promoted by withdrawing one or more medium component(s) that promote(s) growth of undifferentiated cells, or act(s) as an inhibitor of differentiation. Examples of such components include certain growth factors, mitogens, leukocyte inhibitory factor (LIF), and basic fibroblast growth factor (bFGF). Differentiation may also be promoted by adding a medium component that promotes differentiation towards the desired cell lineage, or inhibits the growth of cells with undesired characteristics.

Thus, the method according to the invention may further comprise the culturing of the composition in the presence of a differentiating agent and/or mitotic inhibitors, wherein a differentiation agent for inducing differentiation of the cell or cell line is selected from naturally occurring compounds and synthetic reagents such as retinoic acid, retinoids and derivatives thereof, bone morphogenic proteins, growth factors, trophic factors, macrophage inflammatory proteins, noggin, heparan sulfate, amphiregulin, interleukins and the like. Differentiated cells and cell lines thereof are also subject of the present invention.

The invention further relates to a cell culture system comprising a serum-free medium in accordance with the above-described medium, containing components and a support which allow selectively stem cells to be suspended and/or to exist in a spheroidal form, thereby enriching and separating said stem cells from differentiated and differentiating cells; see also supra. The medium is intended to enable the proliferation of stem cells as well as simultaneously to repress their differentiation. Preferably, the culture medium of the present invention comprises or consist of the components described hereinbefore with reference to the culture method of the present invention. Most preferably, the medium of the present invention has the following composition:

| DMEM, with reduced/without Ca, preferably KO-DMEM (Invitrogen) | |
|---|---|
| Serum replacement Knockout (Invitrogen) | 0.5 - 30 % |
| Non-essential amino acids | 0.1 - 5 % |
| L-glutamine or stable glutamine | 0.1 - 5 % |
| HEPES 1M | 0.1 - 10 % |
| Nucleosides | 0.1 - 5 % |
| 2-mercaptoethanol 50 mM | 0.01 - 0.5 % |
| Antibiotics (e.g. penicillin/streptomycin) | 0.1 - 5 % |
| Antimycotics (e.g. Amphotericin B) | 0.1 - 5 % |
| Growth factors (e.g. basic FGF, FGF-4, TGF-β1, TGF-β2, EGF, IL-6, IL-2, SCF, PDGF-BB, IGF, VEGF) | 0.0001- 30 ng/ml |

Growth factors like for example the FGF family, especially basic FGF and FGF-4, as well as EGF, PDGF-BB, the TGF-β family (especially TGF-β1 and 2), IGF or VEGF, preferably 0.1 - 30 ng/ml, can be used either alone or in combination; see also the examples. For the selection of stem cells, the use of for example firstly only basic FGF is possible. As a result, the cells then proliferate slower, but also more selectively. The growth factors, either alone or combinations thereof accelerating the growth of the cells, cause the expansion of the cells selected according to the described method, depending on the purpose of said cells. In some embodiments, the culture medium further comprises 0.1-100 ng/ml E-cadherin.

One main object of the present invention is the provision of stem cells and cell lines thereof, wherein said cells can also be used for tissue reconstitution or regeneration in a human patient in need thereof. For example, the method of the present invention also comprises the step of administering one or more of the stem cells or one or more of the differentiated cells, obtained by the method of the invention to an individual, for example suffering from a certain medical condition. Those medical conditions may be, for example, a disorder of the sensual nerve system, including those having neurodegenerative disorders, such as Parkinson's disease, multiple sclerosis, Alzheimer's disease, and amyotrophic lateral sclerosis (ALS), Huntington's disease, Down's syndrome, diabetes, cardiac disease, stroke, spinal cord injury, leukemia, aplasia, skin replacement, or hair follicle replacement, wherein said cell(s) are administered to the individual either by injection or implantation.

Though it is expected that the stem cells prepared in accordance with the methods of the present invention are substantially pure populations, it may nevertheless be advisable to asses the extent of possible contamination with undifferentiated cells and the efficiency of differentiation prior to clinical applications. In this context, also microarray systems may be used, for example for determining stem cell markers as well as markers of differentiation into neural, mesodermal, and endodermal phenotypes. Furthermore, candidate genes belonging to families of proteins that are likely to be important in the process of differentiation may be assessed. A corresponding focused microarray to asses human embryonic stem cell differentiation has been described in Yang et al., Stem Cells Dev. 14 (2005), 270-284.

In certain embodiments of the invention, one or more therapeutic agents are administered to the cell(s) prior to administration of said cell(s) to the individual, wherein the therapeutic agent preferably comprises a nucleic acid, a peptide, a polypeptide, a small molecule, or a mixture thereof.

The therapeutic agent is chosen with respect to the purpose it is intended for. For those skilled in the art it does not mean any undue burden to select the corresponding and appropriate therapeutic agents. For example, said therapeutic agent comprises brain-derived neurotrophic factor (BDNF), glial cell-derived neurotrophic factor (GDNF) or IFN-ss, if the individual suffers from multiple sclerosis; BDNF or GDNF, if the individual suffers from Parkinson's disease, or insulin if the individual suffers from diabetes. A review of the further prospects for developmental biology and cell therapy based on embryonic stem cell is provided by Wobus and Boheler, Physiol. Ref. 85 (2005), 635-678.

The cell culture system and stem cells of the present invention are particularly suited for use in drug and toxicity screening as well as for therapeutic applications. For example, stem cells of this invention can be used to screen for factors (such as solvents, small molecules, drugs, peptides, polynucleotides, and the like) or environmental conditions (such as culture conditions or manipulation) that affect the characteristics of the cells, for example during the process of differentiation. Particular screening applications of this invention relate to the testing of pharmaceutical compounds in drug research. It is referred generally to the standard textbook "In vitro Methods in Pharmaceutical Research", Academic Press, 1997, and US patent 5,030,015. Assessment of the activity of candidate pharmaceutical compounds generally involves combining the stem cells or differentiated cells of this invention with the candidate compound, determining any change in the morphology, marker phenotype, or metabolic activity of the cells that is attributable to the compound (compared with untreated cells or cells treated with an inert compound), and then correlating the effect of the compound with the observed change. The screening may be done, for example, either because the compound is designed to have a pharmacological effect on certain cell types, or because a compound designed to have effects elsewhere may have unintended side effects. Two or more drugs can be tested in combination (by combining with the cells either simultaneously or sequentially) to detect possible drug-drug interaction effects. In some applications, compounds are screened initially for potential toxicity (Castell et al., pp. 375-410 in "In vitro Methods in Pharmaceutical Research," Academic Press, 1997). Cytotoxicity can be determined in the first instance by the effect on cell viability, survival, morphology, and expression or release of certain markers, receptors or enzymes. Effects of a drug on chromosomal DNA can be determined by measuring DNA synthesis or repair. [H]thymidine or BrdU incorporation, especially at unscheduled times in the cell cycle, or above the level required for cell replication, is consistent with a drug effect. Unwanted effects can also include unusual rates of sister chromatid exchange, determined by metaphase spread. It is referred to A. Vickers (pp 375-410 in "In vitro Methods in Pharmaceutical Research," Academic Press, 1997) for further elaboration.

Effects of cell function can be assessed using any standard assay to observe for example phenotypes or differentiability, extensive proliferative capacity, self-renewal and maintainance of the stem cell in the cell culture. Where an effect is observed, the concentration of the compound can be titrated to determine the median effective dose.

Thus, in a further embodiment, the present invention relates to a method for screening compounds which affect proliferation, differentiation or survival of stem cells and/or differentiated derivatives comprising preparing stem cells according to the described method of the invention; contacting a sample comprising said stem cells with at least one compound and monitoring the effect of the compound on proliferation, differentiation or survival of stem cells and/or differentiated derivatives comprised within the sample, preferably comprising differentiating cells to generate a composition of neural cell types; contacting with compound(s) and subsequently monitoring the effects of the compound(s) on the ability of the progenitor cells to differentiate into neural cells.

Effects of the test compound on cancer stem cells can also be measured for example by determining the number of cancer stem cells that persist in culture or in the tumors in vivo after treatment with the test compound. In addition to determining the number of cancer stem cells, the effects of the test compound on cancer stem cells' cell-cycle status and marker expression can also be determined by for example flow-cytometry. Furthermore, the effects of the test compounds or biological agents can be identified on the basis of significant differences relative to control cultures with respect to criteria such as the ratios of expressed phenotypes, cell viability, proliferation rate, number of cancer stem cells, cancer stem cell activity upon transplantation in vivo, cancer stem cell activity upon transplantation in culture, cell cycle distribution of tumor cells, and alterations in gene expression.

In a further embodiment, the present invention relates to methods for obtaining and/or profiling a test substance capable of influencing stem cell development and differentiation, comprising the differentiation of stem cells and/or the steps of:
(a) contacting a test sample comprising said stem cells or in vitro differentiated cell with a test substance; and
(b) determining a phenotypic response in said test sample compared to a control sample, wherein a change in the phenotypic response in said test sample compared to the control sample is an indication that said test substance has an effect on cell development and/or tissue structure formation.

Compound(s) for screening, selected from compounds which are intended to have a harmful or beneficial effect on neural cells and which include neurological inhibitors or agents such as, for example, neuroblockers or neurotransmitters and receptors therefore, growth inhibitors or growth factors and receptors therefore and enzymes used in their synthesis, are also comprised by the method of the invention. The test compound may be dissolved in an appropriate solvent, not affecting its properties, those solvents being known to the person skilled in the art, solid, volatile or aerially. The present invention also concerns contact of the cells to radiation, for example in a radiation chamber, this radiation being for example electromagnetic radiation like radio waves, micro waves, infrared radiation, visible light, ultraviolet light, X-rays or γ-rays; particle radiation such as electron beam, neutron radiation, proton radiation and cosmic radiation, either alone or in combination.

Furthermore, the cell culture system of the present invention allows investigation whether a given compound is capable of preventing the onset of a disease like cancer. Therefore, the test compound may be solubilized and added to the culture medium at varying concentrations to determine the effect of the test compounds or agent at each dose. The culture medium may be replenished with the test compound or biological agent at an appropriate time interval in amounts so as to keep the concentration of the agent somewhat constant. This embodiment is particularly useful for identifying and obtaining drugs that can be used as a prophylactic means, which is especially worthwhile considering for the prevention of diseases, especially cancer diseases. This prevention concerns for example "normal" stem cells to become carcinogenic, when treated with a preventing agent and then afterwards being exposed to carcinogenic agents like radiation, viruses or chemicals. Those carcinogenic agents include for example, but are not limited to radiation like UV light, ionizing radiation like X-rays, gamma rays, α-particles, β-particles, protons or neutrons; viruses like the human papilloma virus, hepatitis B, Epstein-Barr virus, human T-cell leukemia virus and Kaposi's sarcoma herpes virus; chemicals like benzene, organic solvents, arsenic, asbestos, vinyl chloride, volatilized chromium and nickel, as well as pollutants and tobacco products. The general mechanism of cancer and its elicitors, as well as detailed cellular processes are described in detail in applicant's co-pending application "Means and methods for the isolation and characterization of cancer stem cells", attorneys docket no. CE18A03/P-EP, filed on the same day as the present application, i.e. on December 01, 2005.

Monitoring the effects on proliferation or viability includes, according to the invention, monitoring the rate of cell proliferation or of cell progeny proliferation, monitoring the nature of cell differentiation and the ratio of differentiated cell types for example ratio of neurons to glia or the like, monitoring symmetric and asymmetric division of (cancer) stem cells, monitoring cell death and the like, monitoring the changes in cell development or morphology, monitoring tumor formation and/or tumor growth, monitoring the changes in expressed phenotypes, amount or type of proteins expressed and the like, monitoring the changes in neuronal characteristics including electrophysiological properties such as resting membrane potential, evoked potential, direction and ionic nature of current flow, dynamics of ion channels, monitoring changes in developmental characteristics like sensitivity towards and/or inducibility of apoptosis, adherence dependency or independency, controlled or uncontrolled cell growth, dependency or independency from other cell contacts, i.e. the "communication" between cells, monitoring up- and down regulation of enzymes and genes, especially growth factors and tumor suppressor genes, and monitoring "behavioral" characteristics like becoming autonomous and leaving united cell structures.

These methods can replace various animal models, and form novel human-based tests and extreme environmental biosensors. In particular, the methods of the invention can be used for toxicological, mutagenic, and/or teratogenic in vitro tests.

For the screening methods of the present invention, the stem cells may be localized in monolayers in culture, in suspension in culture, or affixed to a solid surface. Preferably, the screening method is performed on an array. Arrays for use in the assay of the present invention usually comprise a solid support and attached thereto or suspended thereon the stem cells or in vitro differentiated cells thereof. The use of planar microelectrode arrays for cultured cells and cell aggregates as biosensors is of particular interest. Such arrays generally consist of a substrate of glass, plastic or silicon over which a conductor, e.g. gold, platinum, indium-tin-oxide, iridium, etc., is deposited and patterned. An insulating layer, e.g. photoresist, polyimide, silicon dioxide, silicon nitride, etc., is deposited over the conducting electrodes and interconnects, and then removed in regions over the electrodes to define the recording sites. Cells are cultured directly on this surface and contact the exposed conductor at the deinsulated recording sites. Depending on the size of the electrodes and the cells, recordings of electrical activity can be from a single cell or populations of cells including cell aggregates. Each electrode site is generally connected to the input of a high input impedance, low noise amplifier, with or without AC coupling capacitors, to allow amplification of the relatively small extracellular signals. Examples of such biosensors are described by Novak et al. IEEE Transactions on Biomedical Engineering BME-33(2) (1986), 196-202; Drodge et al., J. Neuroscience Methods 6 (1986), 1583-1592; Eggers et al., Vac. Sci. Technol. B8(6) (1990), 1392-1398; Martinoia et al., J. Neuroscience Methods 48 (1993), 115-121; Maeda et al., J. Neuroscience 15 (1995), 6834-6845; and Mohr et al., Sensors and Actuators B-Chemical 34 (1996), 265-269.

In one preferred embodiment of the present invention, progenitor cells of cells and tissue of the CNS may be analyzed using an electrode array as described above. Means and methods for analyzing regulatory interactions of neuronal activity of cells and tissue cultures on microelectrode arrays are known to the person skilled in the art; see for example van Bergen et al., Brain Res. Protocol 2003/11 (2003), 123-133 and international application WO01/65251. Similarly, cells and tissue related to the liver can be tested; see, e.g., US patent application US2003/0003573.

Preferred compound formulations for testing do not include additional components, such as preservatives that have a significant effect on the overall formulation; see also supra. Thus, preferred formulations consist essentially of a biologically active compound and a physiologically acceptable carrier, e.g., water, ethanol, DMSO, etc. However, if a compound is liquid without an excipient, the formulation may consist essentially of the compound itself. Furthermore, a plurality of assays may be run in parallel with different compound concentrations to obtain a differential response to the various concentrations. As known in the art, determining the effective concentration of a compound typically uses a range of concentrations resulting from 1:10, or other log scale dilutions. The concentrations may be further refined with a second series of dilutions, if necessary. Typically, one of these concentrations serves as a negative control, i.e. at a concentration of zero or below the level of detection.

Compounds and candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. For example, inhibition of tumor-induced angiogenesis and matrix-metalloproteinase expression in confrontation cultures of embryoid bodies and tumor spheroids by plant ingredients used in traditional Chinese medicine has been described by Wartenberg et al. in Lab. Invest. 83 (2003), 87-98.

The test compound may optionally be a combinatorial library for screening a plurality of compounds. Such a collection of test substances can have a diversity of about 10³ to about 10⁵ which is successively reduced in running the method, optionally combined with others twice or more. Compounds identified in the method of the invention, can be further evaluated, detected, cloned, sequenced, and the like, either in solution or after binding to a solid support, by any method usually applied for the detection of a specific DNA sequence such as PCR, oligomer restriction (Saiki et al., Bio/Technology 3 (1985), 1008-1012, allele-specific oligonucleotide (ASO) probe analysis (Conner et al., Proc. Natl. Acad. Sci. USA 80 (1983), 278), oligonucleotide ligation assays (OLAs) (Landegren et al., Science 241 (1988), 1077), and the like. Molecular techniques for DNA analysis have been reviewed (Landegren et al., Science 242 (1988), 229-237). Hence, the method of the present invention can also be used for transcriptional profiling of stem cells and in vitro differentiated cells thereof; see, e.g., Ramalho-Santos et al., Science 298 (2002), 597-600; Tanaka et al., Genome Res. 12 (2002), 1921-1928.

The cell-based assay of the present invention is particularly suited to provide modulation reference patterns, i.e. profiles and databases of modulation reference patterns for a wide range of biologically active compounds. The reference patterns are then used for the identification and classification of test compounds. Evaluation of test compounds may be used to achieve different results.

In one embodiment of the present invention, the above described methods further comprise characterization of stem cells or in vitro differentiated cells thereof, including the measurement of, for example, changes in transcription activity, transcription level, translation activity, translation level, protein expression, protein levels, quantity of, for example, nucleic acids or proteins, processing of nucleic acids (DNA and/or RNA), changes in protein function or activity, alteration in tissue composition or function, changes in gene expression or gene expression levels (especially tumor suppressor genes), changes in activity, intactness and/or functionality, as well as altered quantity of factors such as for example co-factors, transcription factors or growth factors, changes in binding affinities of factors, proteins, enzymes or nucleic acid molecules, up- and/or down-regulation of nucleic acids and/or proteins, changes in metabolism, gain or loss of polymorphisms, altered response to external stimuli or changes in sequence compositions of nucleic acids and/or proteins, which are significantly different from "normal" stem cells. Those changes can be measured using means and methods known to those skilled in the art.

US patent. No. 6,194,158 refers to a diagnostic assay for cancer with a DNA chip of specific sequences for measuring expression levels of certain sequences within a cancer cell to determine whether expression is up- or down-regulated. The DNA chip comprising nucleotide sequences capable of hybridizing to one or more members of a panel of DNA sequences may be synthesized using commonly available techniques. mRNA is isolated from a normal, non-cancer cell as well as from a cancer cell and hybridized to the DNA chip comprising one of more of the sequences from the panel. Hybridization is then detected by any of the available methods known to those skilled in the art. In such a manner, sequences that are either overexpressed or underexpressed in a cancer cell as compared to a normal cell are detected. In a similar manner, mRNA from a cancer cell that has been contacted with a compound may be hybridized to sequences on the DNA chip to determine whether that compound affects expression of a particular sequence. The present invention provides an improvement over this method, in that the "cancer cell" from which mRNA can be isolated is the tumorigenic cancer stem cell of the invention.

In a particularly preferred embodiment, any one of the above-described methods of the present invention comprises the use of a microarray; see also supra. Since most microarray assays are nucleic based assays it is of course understood that the methods of the present invention include the isolation of nucleic acids, preferably mRNA from the stem cells or in vitro differentiated cells and, if necessary, their further processing for making them suitable for the array analysis. Hence, microarrays are particular useful for gene expression profiling of stem cells and include for example Affymetrix GeneChip^{©} microarrays; see for example the recent publication by Maitra et al., Nature Genetics 37 (2005), 1099-1103, in which the characterization of stem cell lines using such microarrays is described. Moreover, current microarray, serial analysis of gene expression (SAGE), and expressed sequence tag (EST) strategies to characterize stem cells and their differentiated derivatives are summarized in Robson, Trends Biotechnol. 22 (2004), 690-612. Gene expression profiling of embryonic stem cells is further described by Rao and Stice in Biology of Reproduction 71 (2004), 1772-1778. In addition, studies of stem cell function using microarray experiments have been performed by Perez-Iratxeta et al. and are described in FEBS Lett. 579 (2005), 1795-1801. In this publication, the authors provide a collection of mRNAs measurements in different cell types and states, and used the results as a source of functional predictions. The data can be accessed online via the internet reference given.

Finally, the unique possibility of combining stem cell technology and the microarray techniques are expected to be useful for regulatory developmental toxicity testing; see for example Bremer and Hartung, Curr. Pharm. Des. 10 (2004), 2733-2747. All these methods may be applied and adapted to the methods of the recent invention.

Furthermore, the present invention relates to screening assays for identifying differentiation-inducing agents in the production of differentiated cells for e.g., cell therapy, which involve any one of the above-described methods for isolating stem cells or the stem cells thereby obtained. For example, assays for screening growth factors, adhesion molecules, immunostimulatory molecules, extracellular matrix components and other materials, alone or in combination, simultaneously or temporally, for the ability to induce directed differentiation of pluripotent and multipotent stem cells are described in international application WO/018760, the disclosure content of which is incorporated herein by reference. In this context, also combinatory libraries in high through put screening may be used, such as combinatorial surface chip compositions for selection, differentiation and propagation of stem cells described in international application WO2004/094602.

The assay methods of the present invention can be carried out in conventional laboratory format or adapted for high throughput screening. The term "high throughput screening" (HTS) refers to an assay design that allows easy analysis of multiple samples simultaneously, and capacity for robotic manipulation. Another desired feature of high throughput assays is an assay design that is optimized to reduce reagent usage, or minimize the number of manipulations in order to achieve the analysis desired. In this context, microfluidic methods may be used as well as screening platforms, wherein the test compound or the stem cells are attached to a solid surface.

Examples of assay formats include 96-well, 384-well or more-well plates, levitating droplets, and "lab on a chip" microchannel chips used for liquid handling experiments. It is well known by those in the art that as miniaturization of plastic molds and liquid handling devices are advanced, or as improved assay devices are designed, that greater numbers of samples may be performed using the design of the present invention. Two or more measurements, optionally at different positions within the array may be taken. Several test substances can be combined and either added simultaneously or sequentially to gain information about possible enhancing or quenching effects. Thus, a further aspect of the invention relates to the method described previously, wherein the contacting step further includes contacting the test sample with at least one second test substance in the presence of said first test substance. Two or more substances tested in combination will provide information about their interaction in general and optionally a compound known to activate or inhibit a disease process may be added to the sample or culture medium.

Furthermore, the above-described methods can, of course, be combined with one or more steps of any of the herein described screening methods or other screening methods well known in the art. Methods for clinical compound discovery comprise for example ultrahigh-throughput screening (Sundberg, Curr. Opin. Biotechnol. 11 (2000), 47-53) for lead identification, structure-based drug design (Verlinde and Hol, Structure 2 (1994), 577-587) and combinatorial chemistry (Salemme et al., Structure 15 (1997), 319-324) for lead optimization. Once a drug has been selected, the method can have the additional step of repeating the method used to perform rational drug design using the modified drug and to assess whether said modified drug displays better affinity according to for example interaction/energy analysis. The method of the present invention may be repeated one or more times such that the diversity of said collection of compounds is successively reduced.

Moreover, the present invention relates to a therapeutic agent, i.e. drug identified or isolated according to the screening methods of the present invention described hereinbefore, which agent hitherto has not been known or used as a therapeutic agent. Hence, the present invention also relates to compositions comprising said agent for use in therapy and diagnosis as well as in diagnostic assays. The drug according to the invention can be combined with suitable diluents or carriers, preferably those which are pharmaceutically acceptable. Examples of such carriers, diluents and methods of formulation may be found in Remington's Pharmaceutical Sciences, 20th ed. (Mack Publishing Co., Easton, PA). To form a pharmaceutically acceptable composition suitable for effective administration, such compositions will contain an effective amount of the modulator. Carriers or diluents are usually sterile and non-toxic, and defined as vehicles commonly used to formulate pharmaceutical compositions for animal or human administration. The diluent is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or non-toxic, non-therapeutic, non-immunogenic stabilizers, and the like. Further examples of suitable pharmaceutical carriers are well known in the art and include phosphate buffered saline solutions, emulsions, such as oil/water emulsions, various types of wetting agents, sterile solutions etc. Compositions comprising such carriers can be formulated by well known conventional methods; see Remington's Pharmaceutical Sciences, supra.

Once a drug has been selected in accordance with any one of the above-described methods of the present invention, the drug or a pro-drug thereof can be synthesized in a therapeutically effective amount. As used herein, the term "a therapeutically effective amount" means the total amount of the drug or pro-drug that is sufficient to show a meaningful patient benefit, i.e. treatment, healing, prevention or amelioration of damaged tissue, or an increase in the rate of treatment, healing, prevention or amelioration of such conditions. In addition or alternatively, in particular with respect to pre-clinical testing of the drug, the term "therapeutically effective amount" includes the total amount of the drug or pro-drug that is sufficient to elicit a physiological response in a non-human animal test.

The appropriate concentration of the therapeutic agent might be dependent on the particular agent. The therapeutically effective dose has to be compared with the toxic concentrations, the clearance rate as well as the metabolic products play a role as to the solubility and the formulation. Therapeutic efficacy and toxicity of compounds can be determined by standard pharmaceutical procedures in cell cultures or experimental animals, e.g., ED50 (the dose therapeutically effective in 50 % of the population) and LD50 (the dose lethal to 50 % of the population). The dose ratio between therapeutic and toxic effects is the therapeutic index, and it can be expressed as the ratio, LD50/ED50.

Depending on the specific conditions being treated, agents may be formulated and administered systemically or locally. Techniques for formulation and administration may be found in Remington's Pharmaceutical Sciences, supra. Other suitable routes may include oral, rectal, transdermal, vaginal, transmucosal, or intestinal administration; parenteral delivery, including intramuscular, subcutaneous, intramedullary injections, as well as intrathecal, direct intraventricular, intravenous, intraperitoneal, intranasal, or intraocular injections, just to name a few.

Furthermore, the present invention relates to the use of a compound identified, isolated and/or produced by any one of the methods of the present invention for the preparation of a composition for the treatment of disorders related to, for example, damaged tissue or aberrant tissue or organ formation, cancer, neurodegenerative disorders, etc.; see also supra and infra. Preferably, the isolated compound or corresponding drugs support general new tissue formation, prevention of tumor formation and/or tumor growth, regeneration of damaged tissue due to chemotherapy, selective removal of cancer stem cells to prevent recurrence of cancer, and/or the selective induced differentiation of cancer stem cells to make the differentiated cells accessible to chemotherapeutic agents.

Additionally, the present invention relates to culture containers and surfaces as described hereinbefore for use in the culture system of the present invention, for example flasks, dishes, multiwell plates, roller bottles, stirred vessels (spinner flasks), bioreactors, or fermentors. In a preferred embodiment of the present invention, said container is pre-filled with a cell culture medium of the present invention. Most preferably, said container is adapted for culturing and isolating adult stem cells and/or tumor stem cells.

The present invention also relates to one or more kits for isolating and/or using the stem cells of the present invention, said kits containing specific reagents such as those described hereinbefore useful for conducting any of the above-described methods of the present invention, comprising the culture medium of the invention, the cell culture system (see supra), or culture media components thereof, growth factors, selectable markers, reference samples, microarrays, chelating substances, culture vessels, cell suspending media, blocking media, washing media and components of said media, vectors, nucleic acid probes and/or a bioreactor. Such a kit would typically comprise a compartmentalized carrier suitable to hold in close confinement at least one container and the compounds of the kit may be sterile, where appropriate. The kit may further include a transfer means, such as pipets, for transferring the suspended cells. In other embodiments, there may be components for application of the stem cells to an individual, such as a syringe, a filter for concentrating the cells, an aqueous solution for suspension of the cells, a needle, and so forth.

The kit may further comprise components for extracting cells from a tissue of interest for the cultivation of the stem cells, such as for example an apparatus for obtaining bone marrow. Examples include syringes, scalpels, and a standard bone marrow aspiration kit of needle and syringe, with trocar containing heparin (commercially available). In embodiments wherein heparin is found to kill a subset of stem cells, there may be a kit with the standard sterile syringe, aspirating needle, stylet, luer-lock adaptor and cleaning rod, etc., without heparin, and so forth. The kit's carrier would further comprise reagents useful for performing said methods and may also contain means for detection such as labeled enzyme, substrates, or the like. Instructions can be provided to detail the use of the components of the kit, such as written instructions, video presentations, or instructions in a format that can be opened on a computer (e.g. a diskette or CD-ROM disk). These instructions indicate, for example, how to use the cells to screen test agents of interest. Most preferably, the instructions refer to the use of the kit in methods concerning the isolation of non-embryonic stem cells, for example adult stem cells and/or tumor stem cells.

As already discussed in context with the assay system of the present invention for screening putative drugs, the observations made in accordance with the present invention can also be applied to establish a novel method of identifying putative target genes for therapeutic intervention within the treatment of a given disease. Instead of developing the identified drug in house, further drug development can also be achieved by a different company. Thus, a further aspect of the present invention relates to a method of conducting a target discovery and stem cell technology business comprising (a) the provision of information on the isolation of stem cells in accordance with the method of the invention as described hereinbefore and/or (b) testing of compounds in accordance with the method of compound screening as described supra; (optionally) conducting therapeutic profiling of therapeutic agents identified in step (a) for efficacy and toxicity in animals; and (c) licensing, to a third party, the rights for further stem cell production and tissue engineering, drug development and/or sales for therapeutic agents identified in step (a) or (b), or analogs thereof.
For suitable lead compounds that have been identified further profiling of the agent, or further analogs thereof, can be carried out for assessing efficacy and toxicity in animals, depending on the modalities of the agreement with the respective third party. Further development of those compounds for use in humans or for veterinary uses will then be conducted by the third party. The subject business method will usually involve either the sale or licensing of the rights to develop said compound but may also be conducted as a service, offered to drug developing companies for a fee.

In addition, the present invention relates to a patient stem cell bank, comprising stem cells obtained by the method of the present invention, wherein said cells are capable of responding to external agents such as drugs and pharmaceuticals for screening, or which are capable of establishing a graft in a recipient and responsive to host environmental signals.

Hence, the method of the invention relates further to the use of stem cells or differentiated cells, as described hereinbefore in a variety of applications including, but not limited to "loss of function" assays of specific genes, "gain of function" assays of exogenous genes, analysis and screening of putative drugs, pharmacological assays, microarray systems, establishment of model systems for pathological cell functions, testing of anti-tumor agents, for investigating differentiation in vitro and/or in vivo, for tissue engineering, for compound testing or screening in pharmaceutical development, for activity and toxicological testing, or therapeutic cell replacement strategies including but not limited to transplantation.

In one embodiment of the invention, further characterization of cancer stem cells comprises the measurement of, for example, changes in transcription activity, transcription level, translation activity, translation level, protein expression, protein levels, quantity of for example nucleic acids or proteins, processing of nucleic acids (DNA and/or RNA), changes in protein function or activity, alteration in tissue composition or function, changes in gene expression or gene expression levels (especially tumor suppressor genes), changes in activity, intactness and/or functionality as well as altered quantity of factors such as for example co-factors, transcription factors or growth factors, changes in binding affinities of factors, proteins, enzymes or nucleic acid molecules, up- and/or down-regulation of nucleic acids and/or proteins; changes in metabolism, gain or loss of polymorphisms, altered response to external stimuli or changes in sequence compositions of nucleic acids and/or proteins which are significantly different from "normal" stem cells. Those changes can be measured using means and methods known to those skilled in the art.

The microarray, as described hereinbefore, further may be used to monitor the progression of disease by assessing and cataloging the differences in gene expression between healthy and cancerous tissues based on the analysis of the changes in patterns of gene expression compared to cancer stem cells from the patient. Thus, cancer can be diagnosed at earlier stages before the patient is symptomatic. The invention can also be used to monitor the efficacy of treatment. For some treatments with known side effects, the microarray is employed to "fine tune" the treatment regimen. A dosage is established that causes a change in genetic expression patterns indicative of successful treatment. Expression patterns associated with undesirable side effects can be avoided. This approach may be more sensitive and rapid than waiting for the patient to show inadequate improvement, or to manifest side effects, before altering the course of treatment.

Alternatively, the "stem cell" qualities of the cancer stem cells of the invention, protein extracts derived from said cells, purified proteins from said cancer stem cells, or proteins derived from the expression of cDNAs from said cancer stem cells (see also genetic modification of cancer stem cells, as described) may be used to induce an immune response in an animal for example. The immune response can be directed against cancer cells, as shown by standard immunological methods. For example, the cancer stem cells (enriched populations of isolated cells) or proteins can be contacted with dendritic cells in culture, antigen presenting cells in culture, or antigen presenting cells and T-cells in culture. Then, antigen-stimulated cells may be infused back into the patient. Thus, the cancer stem cell obtained by the method of the present invention may be further used for the vaccination of animals to get monoclonal antibodies or at least reactive cells.

Furthermore, the cancer stem cells, purified by the method of the present invention, may be used for the establishment of a xenograft model for certain types of cancer. Additionally, animal models which mimic a (cancer) disease, rather than patients, may be used to characterize expression profiles associated with a particular disease or condition. This gene expression data may be useful in diagnosing and monitoring the course of disease in a patient, in determining gene targets for intervention, and in testing novel treatment regimens.

Further applications of stem cells, in particular pluripotent embryonic-like stem cells, preferably derived from teeth are described in international application WO03/066840. Hence, the methods described in this international application for stem cells are also applicable for stem cells, obtainable by the methods of the present invention. Accordingly, methods of reducing clonal stem lines, genetically engineered stem cells, as well as methods for detecting agents capable of modulating the lineage-commitment of lineage uncommitted cells and screening essays are particular included within the scope of the present invention. The same applies to the methods of tissue engineering and the method of treatments described in international application WO03/066840.

As explained above, in traditional cell culture systems, the undifferentiated propagation of stem cells is hampered due to their asymmetric division, wherein starting from one stem cell another stem cell is generated, as well as one Transient Amplifying (TA) cell. In most of the cases, the TA cell exhibits a much stronger proliferation activity as well as an advanced differentiation and therefore has a restricted potential to form different cell types. The faster growing of TA cells leads to overgrowth of the slower proliferating multipotent stem cells and eclipses them. Additionally, the differentiating cells themselves produce further growth factors and signal substances, capable of stimulating the stem cells to differentiate, problems which can be overcome by the present invention.

It is dependent on the cell culture conditions whether the stem cells undergo a symmetric or asymmetric cell division. Surprisingly, it could be shown that asymmetric division of stem cells intended by the present invention can be repressed, if the medium is supplemented with a Wnt-pathway activating or upregulating substance, for example a soluble Wnt-3a factor or a functional part, derivative or analog thereof. Preferably, said Wnt-pathway activating substance comprises E-cadherin or cadherin agonists, and most preferably the cadherin agonist is internalin. Accordingly, the present invention also relates to Wnt-pathway activating or upregulating substances, as defined hereinbefore, for effecting symmetric cell division of stem cells and suppressing the generation of Transient Amplifying (TA) cells.

It will be apparent that the methods, compositions, cell culture media and components thereof as well as the stem cells and their use, as substantially described herein or illustrated in the description and the examples, are also subject of the present invention and claimed herewith. In this respect, it is also understood that the particular culture media compositions, as used in any one of the examples, can be independently used and combined with any one of the embodiments described hereinbefore and claimed in the appendend claims set.

These and other embodiments are disclosed and encompassed by the description and examples of the present invention. Further literature concerning any one of the materials, methods, uses and compounds to be employed in accordance with the present invention may be retrieved from public libraries and databases, using for example electronic devices. For example, the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. Several documents are cited throughout the text of this specification. Full bibliographic citations may be found at the end of the specification immediately preceding the claims. The contents of all cited references (including literature references, issued patents, published patent applications as cited throughout this application and manufacturer's specifications, instructions, etc) are hereby expressly incorporated by reference; however, there is no admission that any document cited is indeed prior art as to the present invention.

A more complete understanding of the present invention, which is generally described in the disclosure above, can be obtained by reference to the following specific examples which are provided herein for purposes of illustration only, and are not intended to limit the scope of the invention.

### EXAMPLES

The examples which follow, further illustrate the invention, but should not be construed to limit the scope of the invention in any way. Detailed descriptions of conventional methods, such as those employed herein can be found in the cited literature; see also "The Merck Manual of Diagnosis and Therapy" Seventeenth Ed. ed by Beers and Berkow (Merck & Co., Inc. 2003).

The practice of the present invention will employ, unless otherwise indicated, conventional techniques of cell biology, cell culture, molecular biology, transgenic biology, microbiology, recombinant DNA, and immunology, which are within the skill of the art.
Methods in molecular genetics and genetic engineering are described generally in the current editions of Molecular Cloning: A Laboratory Manual, (Sambrook et al., (1989) Molecular Cloning: A Laboratory Manual, 2nd ed., Cold Spring Harbor Laboratory Press); DNA Cloning, Volumes I and II (Glover ed., 1985); Oligonucleotide Synthesis (Gait ed., 1984); Nucleic Acid Hybridization (Hames and Higgins eds. 1984); Transcription And Translation (Hames and Higgins eds. 1984); Culture Of Animal Cells (Freshney and Alan, Liss, Inc., 1987); Gene Transfer Vectors for Mammalian Cells (Miller and Calos, eds.); Current Protocols in Molecular Biology and Short Protocols in Molecular Biology, 3rd Edition (Ausubel et al., eds.); and Recombinant DNA Methodology (Wu, ed., Academic Press). Gene Transfer Vectors For Mammalian Cells (Miller and Calos, eds., 1987, Cold Spring Harbor Laboratory); Methods In Enzymology, Vols. 154 and 155 (Wu et al., eds.); Immobilized Cells And Enzymes (IRL Press, 1986); Perbal, A Practical Guide To Molecular Cloning (1984); the treatise, Methods In Enzymology (Academic Press, Inc., N.Y.); Immunochemical Methods In Cell And Molecular Biology (Mayer and Walker, eds., Academic Press, London, 1987); Handbook Of Experimental Immunology, Volumes I-IV (Weir and Blackwell, eds., 1986). Reagents, cloning vectors, and kits for genetic manipulation referred to in this disclosure are available from commercial vendors such as BioRad, Stratagene, Invitrogen, and Clontech. General techniques in cell culture and media collection are outlined in Large Scale Mammalian Cell Culture (Hu et al., Curr. Opin. Biotechnol. 8 (1997), 148); Serum-free Media (Kitano, Biotechnology 17 (1991), 73); Large Scale Mammalian Cell Culture (Curr. Opin. Biotechnol. 2 (1991), 375); and Suspension Culture of Mammalian Cells (Birch et al., Bioprocess Technol. 19 (1990), 251); Extracting information from cDNA arrays, Herzel et al., CHAOS 11, (2001), 98-107.

### Example 1: Enrichment, isolation and differentiation of stem cells of the dental sac

The dental sac is an ectomesenchyme-derived connective tissue sac, surrounding the developing tooth, cells of which are accessible, when wisdom teeth are extracted.

Said cells were obtained by the dissection of the tissue into peaces and incubation of said peaces with a mixture of collagenase and dispase for 20 minutes at 37°C in a shakeable water bath. Afterwards single cells (together with the enzyme) were extracted, put through a cell sieve having a mesh size of 40µm and centrifuged at 230 x g. Single cells were collected while the enzymatic treatment was repeated several times (up to six times) as described above. Collected single cells were transferred into the cell culture system as described in the following:
First of all, a cell culture flask (25cm², Coming) was coated with poly-L-lysine, using 2.5 ml of a 0.1 % poly-L-lysine solution and incubating for 30 minutes at 37°C, followed by a washing step with PBS. Hereafter, the single cells (about 1x10⁶ cells) were seeded into the coated flask together with the medium of the following composition:

| DMEM knockout (Invitrogen) | |
|---|---|
| Serum replacement Knockout (Invitrogen) | 20 % |
| Non-essential amino acids (Invitrogen) | 1 % |
| L-glutamine or stable glutamine | 1 % |
| HEPES 1M (Invitrogen) | 2 % |
| Nucleosides (self produced stock solution) | 1 % |
| 2-mercaptoethanol 50 mM (Invitrogen) | 0.2 % |
| Antibiotics (penicillin/streptomycin; Invitrogen) | 1 % |
| Antimycotics (amphotericin B; Biochrome) | 1 % |
| Growth factors: (basic FGF; R&D) | 10 ng/ml |

Medium was changed every two to three days. In the primary culture various different morphologies of cells were detectable and after two to three weeks first spheres appeared (Fig. 1a), those spheres growing with the time. After one month, those spheres were passaged and seeded into the same cell culture system, whereupon again new spheres developed (Fig. 1b). After a further passage, spheres were extracted, enzymatically dissected in a mixture of trypsin and EDTA and seeded onto matrigel. Cells from different spheres showed a unique, fibroblastidal morphology (Fig. 1c). For differentiation experiments, spheres were enzymatically dissected as described hereinbefore and about 5000 cells were seeded into wells of a 96 well plate (Coming).

Differentiation into different types of cells was induced, as described in the following and images were captured by phase-contrast microscopy.
A) Differentiation into adipocytes was induced, using Dulbecco's Modified Eagle's Medium (DMEM) containing 10 % bovine serum, 1 mM L-glutamine, 100 µg/ml isobutylmethylxhantine, 60 mM indomethacin, 1 µM dexamethasone and 10 µg/ml insulin. After three weeks of incubation, staining was carried out on a globule of fat using Oil Red O, showing the differentiation of sphere-derived cells into adipocytes (Fig. 1d).
B) Differentiation into osteoblasts was induced by using DMEM containing 10 % bovine serum, 1 mM L-glutamine, 10 mM β-glycerophosphate, 0.2 mM ascorbic acid and 0.1 µM dexamethasone. After three weeks of incubation, calcium deposits were detectable by staining the cells with alzarine-red, showing the potential of sphere-derived cells to mineralize and differentiate into osteoblasts (Fig. 1e).
C) Differentiation into neuronal types of cells was induced using all trans-retinoic acid (1 µM added to neurobasal medium containing B27 supplement and 1 % L-glutamine). After a few days, cells showed neuronal morphology (Fig. 1f).

Thus, the cell culture system of the present invention provides an environment where stem cells can be cultivated under conditions, allowing for the development of spheres and where those spheres are differentiable into different cell types.

In other words, from the highly morphological heterogeneous culture, stem cells with a similar morphology have been isolated. Said cells were then differentiated into different cell types, since differentiability is a required characteristic property of stem cells. Thus, the existence of stem cells could be proven. The cultivation of the cells in the cell culture system of the invention as described above could be maintained up to more than seven months, showing the property of self-renewing of the stem cells as well. After seven months, cultivation was discontinued. All of the ten dental sacs which were applied to the cell culture system of the invention developed spheres.

### Example 2: Enrichment, isolation and differentiation of stem cells from teratocarcinoma cell line (NTERA-2 cl.D1, NT2/D1)

NTERA2/cl.D1 cells (pluripotent human embryonic carcinoma cells) were firstly expanded in DMEM containing 4.5 % glucose, 4 mM L-glutamine and 10 % FBS according to the manufacturer's data sheet of ATCC (CRL 1973). A cell culture flask was coated with poly-L-lysine using 2.5 ml of a 0.1 % poly-L-lysine solution and incubated for 30 minutes at 37°C, followed by a washing step with PBS. Afterwards cells were dissected into single cells using a trypsin solution and about 3x10⁵ cells were seeded into the coated flask together with the medium of the following composition:

| DMEM Knockout (Invitrogen) | |
|---|---|
| Serum replacement knockout (Invitrogen) | 20 % |
| Non-essential amino acids (Invitrogen) | 1 % |
| L-glutamine or stable glutamine | 1 % |
| HEPES 1M (Invitrogen) | 2 % |
| Nucleosides (self produced stock solution) | 1 % |
| 2-mercaptoethanol 50 mM (Invitrogen) | 0.2 % |
| growth factor: basic FGF (R&D) | 5 ng/ml |
| growth factor: EGF (R&D) | 1 ng/ml |
| growth factor: TGF-β1 (R&D) | 0.002 ng/ml |
| growth factor: TGF-β2 (R&D) | 0.005 ng/ml |
| growth factor: BMP-4 (R&D) | 0.01 ng/ml |
| growth factor: PDGF-BB (R&D) | 0.5 ng/ml |

Medium was changed at two to three days intervals and after one week first formation of spheres was detectable (Fig. 2a). Spheres were extracted, passaged three times and newly seeded into the same system, whereupon again new spheres were formed (Fig. 2b). The thusly obtained spheres were dissected enzymatically with trypsin and EDTA and seeded onto a 96 well plate to a density of an average of 5000 cells per well.

Differentiation into neuronal cell types was induced by the medium described in the following:

| Neurobasal medium (Invitrogen) | |
|---|---|
| B27 supplement (Invitrogen) | 1:50 |
| L-glutamine (Invitrogen) | 1:100 |
| Insulin-Transferrin-Selenium (ITS, Invitrogen) | 1:100 |
| All-trans retinoic acid (ATRA, Sigma) | 10 ng/ml |
| bFGF (R&D) | 10 ng/ml |
| Forskolin (Sigma) | 1 µM |
| Isobutyl methylxanthine (IBMX, Sigma) | 0.5 mM |
| Laminin (Invitrogen) | 1 mg/ml |

Cells showed neuronal morphology after few days (Fig. 2c, four days after induction).

### Example 3: Maintenance, isolation and differentiation of Human Mesenchymal Stem (HMS) Cells (HMSC)

First of all, a cell culture flask was coated as described in example 1. Afterwards HMS-cells (commercially available; Poietics, Lot. 0F0558) were thawed and seeded into the coated flask to a density of 5000 cells per square centimeter together with the medium described in Example 2. Medium was changed at two to three days intervals and 18 hours after sowings, first dense germs of spheres were detectable (Fig. 3a). After 36 hours incubation in the cell culture system of the invention spheres developed (Fig. 3b and 3c). Spheres were separated, washed with EDTA/HBSS (Hank's balanced salt solution), dissected into single cells using Accumax (PAA) and then seeded into the same cell culture system, whereupon new spheres developed (Fig. 3d).

Thus, it could have been demonstrated that even human mesenchymal stem cells do form spheres, when cultured in the cell culture system of the invention.

### Example 4: Enrichment, isolation and differentiation of stem cells from breast cells isolated from breast milk (HBL-100)

This cell line consists of human, epithelial-like cells considered to date as normal breast cells and serving as a reference for breast cancer investigations. The consideration of said cells as "normal" is not entirely correct, since they contain the SV40 virus genome.
First of all, those cells were expanded in DMEM, 4 mM L-glutamine and 10 % FBS.
After a cell culture flask was coated as described in example 1, about 3x10⁵ HBL-100-cells, after being dissected into single cells by trypsin, were seeded into the coated flasks together with the medium described in Example 2.

Medium was changed at two to three days intervals and after 18 hours of cultivation a lot of adherent cells and some non-adherent cells floating in suspension were detectable (Fig. 4a). Cells in suspension started adhering slightly to each other after 36 hours, and after 60 hours a lot of cells were in suspension (Fig 4b and 4c), which were then passaged into a new culture cup containing identical conditions (first passage). After two days, dense colonies were detectable from which spheres developed (Fig. 4d). Continued cultivation and passaging, wherein the developed spheres were dissected into single cells via trituration with EDTA and DNase, using a round melted Pasteur pipet, resulted in the continued development of new spheres (Fig. 4e). The spheres obtained by this procedure, were dissected using a trypsin solution and seeded onto 96 well plates to a density of an average of 5000 cells per well.

Differentiation into neuronal cell types was induced by the medium described in example 2, and cells showed neuronal morphology after a few days (Fig. 4f and 4g, six days after induction).

### Example 5: Enrichment, isolation and differentiation of stem cells from a breast cancer cell line (MCF-7)

MCF-7 cells, obtained from a bruise of the pleura from an adenocarcinoma of a female breast were expanded in DMEM, 4 mM L-glutamine and 10 % FBS containing penicillin/streptomycin.

After a cell culture flask was coated as described in example 1, about 3x10⁵ HBL-100-cells, after being dissected into single cells by trypsin, were seeded into the coated flasks together with the medium described in Example 2.

Medium was changed at two to three days intervals and after two days of cultivation, the cell culture split up into adherent cells and non-adherent cells, wherein the non-adherent cells developed close-packed and hollow acinaric spheres (Fig. 5a). Additionally, cells developed which possibly excreted globules of fat or vacuoles. After an extended cultivation time an enrichment of dark, massive spheres was detectable (Fig. 5d), and in parallel, on the bottom of the flask, a lot of polynuclear cells developed (Fig. 5b and 5c). This polynuclearity of cells is a characteristic and defect, respectively which is often observed in tumors, but is rarely detectable in cell cultures. Under conditions, provided by the cell culture system of the invention, the MCF-7 cells behave like in a tumor, developing a lot of polynuclear cells (Fig. 5b).

Spheres were passaged three times, dissected into single cells by trituration with EDTA and DNase using a round melted Pasteur pipet, and seeded into the same cell culture system, whereupon again new spheres developed. The spheres obtained by this procedure, were dissected by trypsin and seeded onto 96 well plates to a density of an average of 5000 cells per well.

Differentiation into neuronal cell types was induced by the medium described in example 2, and cells showed neuronal morphology after a few days (Fig. 5e and 5f, 3 days, e.g. 1 week after induction).

### Example 6: Enrichment, isolation and differentiation of stem cells from ovarian carcinoma

Ovarian carcinoma tissue was dissected into peaces and incubated with a mixture of collagenase and dispase for 20 minutes at 37°C in a shakeable water bath. Afterwards single cells (together with the enzyme) were extracted, put through a cell sieve having a mesh size of 40 µm and centrifuged at 230 g. Single cells were collected while the enzymatic treatment was repeated several times (six times) as described above. Collected single cells were transferred into the cell culture system as described in the following:

First of all, the cell culture flask was coated as described in example 1. Afterwards the single cells (1x10⁶ cells) were seeded into the coated flasks together with the medium described in Example 2.

At first, in the primary culture various different morphologies were detectable. The existence of polynuclear cells which did not proceed to proliferate, was striking (Fig. 6a). Medium was changed every two to three days and after two to three weeks, first spheres were detectable (see Fig. 6a), further growing with the time. After one month, those spheres were passaged, dissected into single cells by trituration with EDTA and DNase using a round melted Pasteur pipet, and seeded into the same cell culture system, whereupon again new spheres developed (Fig. 6b). Sometimes, "flakes" of small stem cells adhered to a big differentiated cell, as shown in Fig. 6c. After six weeks, a strong expansion of those cells, as well as proliferation of the spheres was detectable.

It will be recognized that the compositions and procedures provided in the description can be effectively modified by those skilled in the art without departing from the spirit of the invention embodied in the claims that follow.

## Claims

1. A method for the enrichment of stem cells in a suspension of a plurality of cells, comprising culturing said plurality of cells in a first container comprising a culture medium under conditions allowing stem cells substantially in suspension and separating stem cells from non-proliferating cells by passaging the suspension into a second container, thereby enriching the stem cells, wherein said method preferably does not comprise the step of co-cultivation with feeder cells, and wherein said culture medium is preferably substantially serum-free.

2. The method of claim 1, wherein the culture medium comprises KNOCKOUT-Dulbecco's modified Eagle's medium (KO-DMEM), and preferably has a low calcium content.

3. The method of any one of claims 1 or 2, wherein the culture medium comprises Fibroblast Growth Factor-4 (FGF-4), Tumor Growth Factor-beta (TGF-β), and/or Bone Morphogenetic Protein-4 (BMP-4).

4. The method of any one of claims 1 to 3, wherein the culture medium comprises a Wnt-pathway activating substance, preferably E-cadherin or cadherin agonists, wherein said cadherin agonist is internalin, or a Wnt-pathway upregulating substance, preferably a soluble Wnt3a factor or a functional part, derivative or analog thereof.

5. The method of any one of claims 1 to 4, comprising a step of culturing the cells under conditions preventing adherence of stem cells and non-proliferating cells to the surface of the container, for example wherein adherence of the cells is blocked by a blocking substance, which is selected from the group consisting of human serum albumin, animal serum albumin, gelatin, Ficoll 70, Ficoll 400, hydrogel, teflon and nanoparticles.

6. The method of any one of claims 1 to 5, wherein the surface of at least one container is coated with a substrate to be cleaved by a protease specifically expressed by stem cells at an enhanced level compared to non-proliferating cells.

7. The method of claim 6, wherein the container is coated with poly-L-amino acids, preferably poly-L-lysine and/or poly-L-ornithine.

8. The method of any one of claims 1 to 7, comprising culturing the cells under conditions, allowing spheroid formation of the stem cells.

9. The method of any one of claims 1 to 8, comprising culturing the cells under hypoxic conditions, for example wherein said hypoxic conditions are provided by the use of a controlled incubator or bioreactor.

10. The method of any one of claims 1 to 9, wherein said stem cells are non-embryonic stem cells, preferably adult stem cells, or tumor stem cells.

11. A population of stem cells or spheroids obtained by the method of any one of claims 1 to 10.

12. A method of producing in vitro differentiated cells, comprising contacting stem cells or spheroids of claim 11 with a differentiation agent and preparing a composition, comprising differentiated cells.

13. A stem cell culture medium as defined in any one of claims 1 to 10.

14. The culture medium of claim 13 comprising:
| Knockout-DMEM | |
|---|---|
| Serum Replacement Knockout | 0,5-30% |
| Non-essential amino acids | 0,1-5 % |
| L-glutamine or stable glutamine | 0,1-5 % |
| HEPES (1M) | 0,1-10% |
| Nucleosides | 0,1-5 % |
| 2-mercaptoethanol (50 mM) | 0,01-0,5% |
| antibiotics (optionally) | 0,1-5 % |
| antimycotics (optionally) | 0,1-5 % |
| growth factors | 0,0001-30 ng/ml |

15. A cell culture system, comprising a serum-free medium in accordance with any one of claims 13 and 14, containing components and a support which selectively allow stem cells to be suspended and/or in a spherical form, thereby enriching and separating said stem cells from differentiated and differentiating cells.

16. A method for screening compounds which affect proliferation, differentiation or survival of stem cells and/or differentiated derivatives, comprising contacting a sample comprising a stem cell of claim 11 or a differentiated cell obtained by the method of claim 12 with at least one compound to be screened, and monitoring the effect of the compound on proliferation, differentiation or survival of stem cells and/or differentiated derivatives comprised within the sample.

17. A therapeutic agent, identified or isolated according to the method of claim 16, which agent hitherto has not been known or used as a therapeutic agent.

18. A pharmaceutical composition comprising the therapeutic agent of claim 17, and optionally a pharmaceutically acceptable carrier.

19. Kit for use in the method of any one of claims 1 to 10, 12 or 16, comprising a culture medium of claims 13 or 14, a cell culture system of claim 15 or culture media components thereof, growth factors, selectable markers, reference samples, microarrays, chelating substances, cell suspending media, blocking media, washing media, components of said media, container, vectors, and/or nucleic acid probes.

20. Use of a stem cell of claim 11 or a differentiated cell obtained by the method of claim 12 or the method of any one of claims 1 to 10, 12 or 16 for loss of function assays of specific genes, gain of function assays of exogenous genes, analysis and screening of putative drugs, pharmacological assays, microarray systems, establishment of model systems for pathological cell functions, testing of anti-tumor agents, for investigating differentiation in vitro and/or in vivo, for tissue engineering, for compound testing or screening in pharmaceutical development, for activity or toxicological testing or therapeutic cell replacement strategies, including but not limited to transplantation.
